# EUROPÄISCHE PATENTSCHRIFT

(11) **EP 1 644 737 B1**
(45) Veröffentlichungstag und Bekanntmachung des Hinweises auf die Patenterteilung: **08.10.2008**
(21) Anmeldenummer: 04763136.1
(22) Anmeldetag: 08.07.2004
(51) Int. Cl.: G01N 33/558, G01N 33/80

(54) **VORRICHTUNG UND VERFAHREN ZUR SIMULTANEN DURCHFÜHRUNG VON BLUTGRUPPENBESTIMMUNG, SERUMGEGENPROBE UND ANTIKÖRPERSUCH-TEST**
DEVICE AND METHOD FOR SIMULTANEOUS CARRYING OUT OF BLOOD GROUP DETERMINATION, SERUM CROSS-CHECK AND ANTIBODY DETECTION TEST
DISPOSITIF ET PROCEDE POUR REALISER SIMULTANEMENT UNE DETERMINATION DE GROUPE SANGUIN, UNE CONTRE-EPREUVE SERIQUE ET UN TEST DE DETECTION DES ANTICORPS

(30) Priorität: 09.07.2003 DE 10330981
(43) Veröffentlichungstag der Anmeldung: 12.04.2006
(73) Patentinhaber: Medion Diagnostics GmbH, 3186 Düdingen (CH)
(72) Erfinder: SCHWIND, Peter, 1700 Fribourg (CH); LÖSTER, Klemens, 16562 Bergfelde (DE)
(74) Vertreter: Bublak, Wolfgang
(86) Internationale Anmeldenummer: PCT/EP2004/007525
(87) Internationale Veröffentlichungsnummer: WO 2005/005986

(56) Entgegenhaltungen:
- WO-A-88/03650
- WO-A-94/29696
- WO-A-97/31268
- WO-A-97/34148
- US-A- 4 943 522
- US-A1- 2002 110 803
- US-A1- 2003 040 021
- US-A1- 2003 045 001
- US-B1- 6 203 757
- US-B1- 6 372 515

## Beschreibung

Die Erfindung betrifft eine Vorrichtung für Lateral-Diagonal-Fluss Multiparameter Tests, insbesondere auf den Gebieten der Immunhämatologie und der Infektionsserologie, zum gleichzeitigen, qualitativen oder quantitativen Bestimmen mehrerer Analyten, wobei wenigstens ein Analyt ein zellulär-gebundener Analyt ist, in einer flüssigen Probe, wobei für die Bestimmung mindestens zwei Sorten Indikatorpartikel verwendet werden, von denen mindestens eine Sorte Erythrozyten sind, umfassend eine Aufgabezone zum Auftragen der flüssigen Probe, eine zum Eindringen von zellulären Komponenten geeignete poröse Membran mit mindestens zwei Indikatorzonen auf der Membran, die mit dem/den Analyten in Wechselwirkung treten können und mindestens einem Absorptionsbereich auf der Membran, welcher die Flüssigkeit nach Passieren der Indikatorzonen aufnimmt, wobei die Indikatorzonen zwischen der Aufgabezone und einem Absorptionsbereich liegen, wobei die Fließrichtungen von der Aufgabezone durch die jeweiligen Indikatorzonen zu einem Absorptionsbereich (Fließspuren) im Wesentlichen parallel sind und mindestens zwei unterschiedliche Fließspuren vorliegen, wobei die Membran eine erste Indikatorzone umfasst, die ein Bindungselement zur Bindung eines zellulär-gebundenen Analyten enthält und die Membran eine zweite Indikatorzone umfasst, die ein Bindungselement zur Bindung eines im Plasma enthaltenen Analyten enthält.

Die Erfindung betrifft weiter ein Verfahren zur Bestimmung mehrerer Analyten in einer flüssigen Probe, umfassend das Auftragen der Probe auf die Aufgabezone einer Membran der erfindungsgemäßen Vorrichtung, wobei diese Probe in ausreichender Menge vorliegt, um die Probenflüssigkeit dazu zu veranlassen, in Richtung Absorptionsbereich durch die Indikatorzonen zu fließen und um die Analyten oder ihre Derivate in der Probenflüssigkeit dazu zu veranlassen, in den Indikatorzonen einen Komplex zu bilden, insbesondere zur simultanen Bestimmung von zellulären und plasmatischen Parametern, vorzugsweise zur simultanen Durchführung von Blutgruppenbestimmung und Serumgegenprobe und/oder Antikörpersuch-Test sowie zur simultanen Durchführung von Blutgruppenbestimmung und Bestimmung transfusionsrelevanter infektionsserologischer Marker sowie zur simultanen Durchführung von Blutgruppenbestimmung und Bestimmung von Antikörpern, die gegen andere Blutzellen als Erythrozyten gerichtet sind, insbesondere anti-Thrombozyten und anti-Lymphozyten Antikörper, und wobei mindestens zwei sorten indikatorpartikel verwendet werden, von denen mindestens eine sorte erythrozyten sind.

Um Komplikationsrisiken z. B. bei einer Transfusion, insbesondere Blutgruppenunverträglichkeiten, viralen und/oder bakteriellen Kontaminationen, vorzubeugen, werden bekanntermaßen verschiedene Labortests am Spender- und Patientenblut vorgenommen zur Bereitstellung von Komponenten, die mit dem Rezipienten blutgruppenserologisch kompatibel sind und frei von bekannten übertragbaren Pathogenen sind. Die jeweiligen serologischen Tests beinhalten in der Regel die Blutgruppenbestimmung beim Spender und Empfänger, insbesondere von Blutgruppen der Blutgruppensysteme AB0, Rh und Kell, Serumgegenprobe beim Spender und Empfänger, Antikörpersuche nach irregulären Antikörpern, beim Spender und Empfänger sowie Antikörperidentifizierung beim Empfänger bei Vorliegen irregulärer Antikörper. Der Nachweis von Antikörpern gegen Thrombozyten und/oder Lymphozyten wird im Zusammenhang mit Transfusionen und Transplantationen ebenfalls durchgeführt.

Infektionsserologische Tests am Spender umfassen bekanntermaßen die routinemäßige Bestimmung von Antikörpern insbesondere gegen HIV-1, HIV-2, gegen HCV, gegen *Treponema pallidum* (Syphilis), sowie die Bestimmung des Hepatitis B Oberflächen-Antigens (= HbsAg: Hepatitis surface Antigen).

WO 97/31268 offenbart einen chromatographischen Streifen zur Durchführung eines Bindungsassays zum qualitativen oder quantitativen Bestimmen eines Analyten in einer Probe. US 4,943,522 offenbart ein Verfahren und eine Einrichtung zur Durchführung spezifischer Bindung-Paar-Assays, wie z.B. Immunassays.

In der **blutgruppenserologischen Diagnostik** werden allgemein Parameter nachgewiesen, die besonders im Zusammenhang mit Transfusionen bzw. dem Morbus Hämolyticus Neonatorum (Mhn) von Bedeutung sind. Dabei handelt es sich unter anderem um den Nachweis von Antigenen auf der Oberfläche der Erythrozyten, die für die Blutgruppen charakteristisch sind (Blutgruppenbestimmung). Weitere wichtige Antigensysteme befinden sich auch auf Thrombozyten, Granulozyten, Lymphozyten, die ebenfalls bei Transfusion und Transplantation eine Rolle spielen. Bei Thrombozyten-und Granulozyten-Antigenungleichheit zwischen Mutter und Fötus kann es zu Mhn vergleichbaren Krankheitsbildern am Neugeborenen kommen. Ferner handelt es sich um den Nachweis von regulären Blutgruppen-Antikörpern (Isoagglutinine) und um den Nachweis von irregulären Blutgruppen-Antikörpern im Serum oder Plasma.

Die **Isoagglutinine** oder **regulären Antikörper** werden von allen Menschen frühzeitig nach der Geburt erworben und korrespondieren zur jeweiligen Blutgruppe des AB0-Systems. Sie sind gegen diejenigen Blutgruppenantigene A bzw. B, die dem Individuum selbst fehlen, gerichtet, d. h. Personen mit Blutgruppe A haben anti-B, Personen mit Blutgruppe B haben anti-A, Personen mit Blutgruppe 0 haben anti-A und anti-B; Personen mit Blutgruppe AB haben keine Isoagglutinine. Die regulären Antikörper werden auch "komplett" genannt, weil sie Erythrozyten im NaCl-Milieu direkt agglutinieren können.
Die **irregulären- oder Alloantikörper** werden im Gegensatz zu den Isoagglutininen durch spätere Immunisierungen erworben, v. a. durch Transfusion oder Schwangerschaft. Deshalb haben die meisten Menschen keine irregulären Blutgruppenantikörper. Die transfusionsrelevanten irregulären Antikörper sind in der Regel wärmereaktiv und gehören überwiegend der IgG-Klasse an. Sie sind im Gegensatz zu den regulären Antikörpern im NaCl-Milieu nicht in der Lage, Erythrozyten direkt zu agglutinieren.

Bekanntermaßen werden zur **Blutgruppenbestimmung** die Erythrozyten der zu testenden Person (Spender oder Empfänger) mit Reagenzien, welche blutgruppenspezifische Antikörper enthalten, zusammengebracht. Üblicherweise handelt es sich um Flüssigkeitstests, bei denen durch Mischen einer Erythrozyten-haltigen Probe mit einer Probe, welche Antikörper enthält, die gegen ein bestimmtes Blutgruppenmerkmal gerichtet sind, ein Testansatz hergestellt wird. Der Testansatz wird dann über einen definierten Zeitraum und unter definierten Bedingungen inkubiert und nach Abschluss der Inkubation oder direkt oder nach einem Zentrifugationsschritt entweder visuell oder mit optischen Methoden auf eine eventuelle Agglutination oder Adsorption der Erythrozyten überprüft. Die vorherrschende Endpunktmessung in der Blutgruppenserologie ist nach wie vor die Hämagglutination. Für jede zu bestimmende Blutgruppe muss ein eigener Ansatz pipettiert werden, d. h. z. B. die Bestimmung der 9 wichtigsten Blutgruppen A, B, D, C, c, E, e, Cw und K erfordert ohne Kontrolle 9 getrennte Ansätze.

Für die **Serumgegenprobe** werden bekanntermaßen Zellreagenzien mit bekannter AB0-Blutgruppe (A1, A2, B, 0) verwendet, welche mit dem Serum oder Plasma der zu testenden Person inkubiert werden. Nach einem Zentrifugationsschritt wird visuell bzw. mit optischen Methoden auf eine eventuelle Agglutination der Erythrozyten hin überprüft. Für eine Serumgegenprobe mit den 4 genannten Testzellen müssen herkömmlicherweise 4 Ansätze pipettiert werden.

Für die **Suche** nach **irregulären Antikörpern** werden bekanntermaßen Panel bestehend aus normalerweise 2 oder 3 Blutgruppe 0-Zellen verwendet, deren kombiniertes Antigenprofil die wichtigsten Antigene, insbesondere der Blutgruppensysteme Rh, Kell, Duffy, Kidd, MNS, P, Lewis, Lutheran enthält. Das Zellreagenz wird mit dem Serum oder Plasma der zu testenden Person zusammengebracht, inkubiert und nach einem Zentrifugationsschritt visuell bzw. mit optischen Methoden auf eine eventuelle Agglutination der Erythrozyten überprüft. Für die Bestimmung einer Patientenprobe müssen 2 bis 3 Ansätze pipettiert werden.

Für die **Identifikation** der **irregulären Antikörper,** die in der Regel nach einem positiven Antikörper-Suchtest erfolgt, werden Panel bestehend aus bis zu 16 Blutgruppe 0-Zellen verwendet, deren Antigenprofile die wichtigsten Antigene, insbesondere der Blutgruppensysteme Rh, Kell, Duffy, Kidd, MNS, P, Lewis, Lutheran, in genau abgestimmter Weise abdecken. Das Zellreagenz wird mit dem Serum oder Plasma der zu testenden Person zusammengebracht, inkubiert und visuell bzw. mit optischen Methoden auf eine eventuelle Agglutination der Erythrozyten überprüft. Für die Bestimmung einer Patientenprobe müssen bis zu 16 Ansätze pipettiert werden.
Da die meisten transfusionsrelevanten irregulären Antikörper vom IgG-Typ und deshalb inkomplett sind, müssen die für die Antikörpersuche und-identifikation beschriebenen Reaktionen in der Regel verstärkt werden, um den Endpunkt Hämagglutination nachweisen zu können. Gebräuchlichstes Reagenz ist hierbei ein polyklonales anti-Humanglobulin-Antikörperreagenz, dem häufig anti-Komplement Antikörper zugesetzt sind (typischerweise anti-C3d und/oder anti-C3b).

Eine gebräuchliche Methode zum Nachweis von Thrombozyten-Antikörpern ist der sogenannte MAIPA-Test (Monoclonal Antibody Immobilisation of Platelet Antigens). Hierbei werden Test-Thrombozyten mit dem zu testenden Serum inkubiert. Nach einem Waschschritt wird mit einem monoklonalen z. B. Maus-Antikörper, der für ein bestimmtes Thrombozyten-Glykoprotein spezifisch ist, inkubiert. Die Thrombozyten werden daraufhin lysiert und das verdünnte Lysat wird in ein mit z. B. Ziege anti-Maus Antikörpern beschichtetes Reaktionsgefäss einer Mikrotiterplatte gegeben. Der Ziege anti-Maus Antikörper bindet den Maus-Antikörper und den daran befindlichen Thrombozytenglykoprotein-Humanantikörper-Komplex. Der humane Antikörper wird durch Zugabe eines Enzym konjugierten Ziege anti-human IgG nachgewiesen.

Mit herkömmlichen diagnostischen Tests können nur entweder zelluläre oder plasmatische Parameter bestimmt werden. Zur Bestimmung von Blutbestandteilen müssen grundsätzlich zuvor Zellen und Plasma getrennt werden.

**Lateral-Fluss-Tests** finden heute vielfach Anwendung als Schnelltests, z. B. als Schwangerschaftstests, zur Bestimmung von Infektionsmarkern oder als Drogenscreen. Eine Lateral-Fluss-Test Anordnung besteht bekanntermaßen aus einem festen Träger, auf dem eine Aufgabezone für die zu untersuchende Probe aufgebracht ist, eine Trennmembran, auf der Bindungselemente, z. B. Fängerantikörper bzw. -antigene gebunden sind und auf der sich Bindungsreaktionen nachweisen lassen und ein saugfähiger Absorptionsbereich, der die zu untersuchende Probe linear durch die Trennmembran fließen lässt.

Testmembranen herkömmlicher Lateral-Fluss-Tests werden in der Regel mit chromatographie-ähnlicher Auftrennung beschrieben. Der Analyt in der Probe bindet spezifisch an die in einer Membran befestigten Bindungselemente, die in der Regel in hintereinanderliegenden bzw. übereinanderstehenden Banden angeordnet als Indikatorzonen vorliegen. Der Bindungskomplex wird durch Indikatorpartikel sichtbar gemacht, welche in der Regel in einem Konjugat-Freisetzungs-Pad eingetrocknet in der Anordnung bereits vorliegen. Das Konjugat-Freisetzungs-Pad ist zwischen Aufgabezone und Membran angebracht. Die vorbeschichteten farbigen Indikatorpartikel sind beispielsweise mit einem gegen den gesuchten Analyten gerichteten Antikörper beschichtet.

Das übliche Lateral-Fluss-Test-Format ist das eines sogenannten "Sandwich-Assays", bei dem sowohl die Indikatorzone als auch die Indikatorpartikel mit gegen den gesuchten Analyten gerichteten Liganden, normalerweise ein Antikörpern, belegt sind. Dabei ist der Ligand (Bindungselement) an die Membran immobilisiert. Das Detektor-Reagenz, normalerweise ein Antikörper, welcher an gefärbte Polystyrol-Partikel oder kolloidale Metalle gebunden ist, ist im Konjugat-Freisetzungs-Pad auswaschbar deponiert. Dieser Bindungskomplex dient als Indikatorpartikel. Nach Auftragen der zu untersuchenden Probe benetzt diese sehr schnell das Konjugat-Freisetzungs-Pad, wodurch die Indikatorpartikel mobilisiert werden. Die Indikatorpartikel migrieren mit der Flüssigkeitsfront entlang der porösen Membran. Ein in der Probe befindlicher Analyt wird durch den Antikörper, der an das Indikatorpartikel gekoppelt ist, gebunden. Wenn die Probe die Indikatorzone passiert, wird der Analyt/Indikatorpartikel-Komplex in der Indikatorzone durch Reaktion des Analyten mit dem in der Indikatorzone gebundenen Antikörper immobilisiert, was zu einem sichtbaren Signal führt.

Ein weiteres bekanntes Testformat für kleine Analyten mit nur einer einzigen antigenen Determinante, die nicht gleichzeitig zwei Antikörper binden kann, ist der sogenannte "Kompetitions-Assay". Das an die Indikatorpartikel gebundene Detektor-Reagenz ist normalerweise ein dem Analyten identisches oder analoges Molekül. Die Indikatorpartikel sind im Konjugat-Freisetzungs-Pad deponiert. Die Indikatorpartikel migrieren mit der Flüssigkeitsfront entlang der porösen Membran. Wenn die Probe, die Analyt enthält, und die Indikatorpartikel (die effektiv ebenfalls Analyt enthalten) die Indikatorzone passieren, bindet ein Teil der Analytmoleküle in der Probe und ein Teil der Indikatorpartikel. Je mehr Analyt sich in der Probe befindet, umso effektiver wird er mit der Bindung der Indikatorpartikel kompetieren, umso schwächer wird das Signal.

Bekanntermaßen sind diese Indikatorpartikel überwiegend aus kolloidalem Gold oder aus Polystyrol, die mit dem Fachmann bekannten Methoden hergestellt und beschichtet werden. In den typischen Lateral-Fluss-Tests Formaten werden die Analyten indirekt bestimmt. Unter direkter Bestimmung eines Analyten wird hier verstanden, dass der Analyt bereits an das Indikatorpartikel (z. B. Erythrozyt) natürlich gebunden ist. In dem gebräuchlicheren Fall der indirekten Bestimmung des Analyten enthält die zu testende Probe in der Regel eine nicht zellulär gebundene, z. B. plasmatische Komponente als Analyten und es werden neben der zu testenden Probe zwei Reagenz-Komponenten benötigt, nämlich Indikatorpartikel und Bindungselement. Bei der indirekten Bestimmung bindet der Analyt zunächst an die aus dem Konjugat-Freisetzungs-Pad herausgelösten Indikator-Partikel, bevor dieser Komplex dann durch eine zweite Reaktion mit dem Bindungselement in den Indikatorzonen immobilisiert wird.

Bei der **Verwendung herkömmlicher Lateral-Fluss-Tests mit Erythrozyten** als Indikatorpartikeln, die die zu bestimmenden Analyten, beispielsweise Blutgruppen-spezifische Antigene, gebunden haben, werden bislang in den Indikatorzonen Antikörper gegen korrespondierende Blutgruppenantigene als Bindungselemente in hintereinanderliegenden bzw. übereinanderstehenden Banden nur einer Fließspur angeordnet, wie zum Beispiel anti-A, anti-B gegen die Blutgruppen-Antigene A bzw. B oder Antikörper gegen Antigene des Rh Blutgruppensystems. Dabei weisen herkömmliche Lateral-Fluss-Tests den Nachteil auf, dass die an die Antikörper gebundenen Erythrozyten eine Flussbarriere für die weiter zu untersuchenden Analyte, beispielsweise weitere Zell-assoziierte Antigene, in einer Probe bilden. Durch Agglutination oder Adsorption von Zellen in einer proximal zur Aufgabezone liegenden Bande von Bindungselementen können sich weitere Analyten, insbesondere Zellen bzw. Zellfragmente, in der zu untersuchenden Probe nicht weiter ungehemmt und sichtbar auftrennen und können folglich nicht eindeutig bzw. vollständig nachgewiesen werden. Dies kann z. B. bei einer Person, die Blutgruppe AB Rh D positiv ist, zu einer Abschwächung bzw. Eliminierung der B- und der D-Bande führen, was zu einer Fehlinterpretation im Sinne von Blutgruppe A Rh negativ führen könnte. Bislang konnten deshalb speziell in der blutgruppenserologischen Diagnostik keine Lateral-Fluss-Test mit mehr als einer Indikatorzone angewendet werden. Für die Messung mehrerer, insbesondere zellulärer und plasmatischer Blutgruppenparameter müssen bislang Einzelparameter-Tests separat durchgeführt werden.

WO97/31268 und US6,203,757 offenbaren jeweils Vorrichtungen zum gleichzeitigen, qualitativen oder quantitativen Bestimmen mehrerer Analyten.

**Aufgabe** der Erfindung ist es, die im Hinblick auf den Stand der Technik angeführten Nachteile, insbesondere die der hintereinanderliegenden bzw. überlagernden Indikator- bzw. Nachweiszonen herkömmlicher Lateral-Fluss-Tests, für eine gleichzeitige Messung verschiedener Proben-Parameter, insbesondere von zellulären und plasmatischen Parametern, zu überwinden.

Die **Aufgabe wird erfindungsgemäß gelöst** zum einen **durch eine Vorrichtung** zum gleichzeitigen, qualitativen oder quantitativen Bestimmen eines oder mehrerer Analyten, wobei wenigstens ein Analyt ein zellulär-gebundener Analyt ist, in einer flüssigen Probe, wobei für die Bestimmung mindestens zwei Sorten Indikatorpartikel verwendet werden, von denen mindestens eine Sorte Erythrozyten ist, umfassend eine Aufgabezone zum Auftragen der flüssigen Probe, eine zum Eindringen von zellulären Komponenten geeignete poröse Membran mit mindestens zwei Indikatorzonen auf der Membran, die mit dem/den Analyten in Wechselwirkung treten können und mindestens einem Absorptionsbereich auf der Membran, welcher die Flüssigkeit nach Passieren der Indikatorzonen aufnimmt, wobei die Indikatorzonen zwischen der Aufgabezone und dem Absorptionsbereich liegen, dadurch gekennzeichnet, dass die Fließrichtungen von der Aufgabezone durch die jeweiligen Indikatorzonen einer Gruppe zu einem Absorptionsbereich, welche Fließspuren darstellen, im Wesentlichen parallel sind und mindestens zwei unterschiedliche Fließspuren vorliegen, wobei die Membran eine erste Indikatorzone umfasst, die ein Bindungselement zur Bindung eines zellulär-gebundenen Analyten enthält und die Membran eine zweite Indikatorzone umfasst, die ein Bindungselement zur Bindung eines im Plasma enthaltenen Analyten enthält.

Die **Indikatorzonen** der erfindungsgemäßen Vorrichtung befinden sich auf der Membran und umfassen Bindungselemente, die die zu bestimmenden Analyte in der Probe abfangen bzw. binden. In den Indikatorzonen werden die Bindungsreaktionen zwischen Analyt und Bindungselement nachgewiesen. Als besonders bevorzugte Bindungselemente werden Antikörper bzw. Antikörperfragmente, Lektine, Antigene bzw. Antigen-Epitope und/oder Zellen bzw. Zellfragmente an der porösen Membran angebracht. Die Indikatorzonen umfassen vorzugsweise jeweils ein Bindungselement gegen einen zu untersuchenden Analyten.

In einer **Ausführungsform der Erfindung** sind die Indikatorzonen so angeordnet, dass die Probenflüssigkeit pro Fließspur **nicht mehr als eine Indikatorzone durchströmt.** Beispielhaft sind die Indikatorzonen versetzt auf der Membran angeordnet. Die Anordnung der Indikatorzonen ist dabei vorzugsweise in einer von proximal nach distal oder umgekehrt diagonal verlaufenden Reihe ausgestaltet. Besondere Ausführungsformen sind V-förmig, W-, M-, oder N-förmig oder umgekehrt V-förmig, W-, M-, oder N-förmig ausgestaltet. In einer weiteren Ausführungsform sind die Indikatorzonen parallel nebeneinander versetzt in einer linearen Reihe angeordnet.
Die Einführung versetzter Indikatorzonen macht eine Multiparametertestung mit Erythrozyten als Indikatorpartikeln in einer lateralen Anordnung erst möglich. Die besonders bevorzugte Ausführungsform einer diagonalen Anordnung hat den Vorteil, dass die Bezeichnung der Ergebnisse besonders praktisch und leicht ablesbar auf die erfindungsgemäße Anordnung aufgebracht werden kann, da jeder nachzuweisende Parameter eine definierte X- und Y-Position aufweist, betrachtet man die Anordnung der erfindungsgemäßen Vorrichtung als ein Koordinatensystem mit Ordinate (Ebene der Fließrichtung) und Abszisse (Ebene der Auftragszone).

In einer weiteren **Ausführungsform** der Erfindung sind **mehr als eine solche Reihe** von Indikatorzonen, vorzugsweise jeweils von proximal nach distal oder umgekehrt diagonal verlaufend, oder beispielsweise auch V-förmig, W-, M-, oder N-förmig oder umgekehrt V-förmig, W-, M-, oder N-förmig verlaufend, in Fließrichtung hintereinander und/oder seitlich versetzt angeordnet und die Indikatorzonen der verschiedenen Reihen entweder zueinander **auf Lücke** angeordnet, so dass die Probenflüssigkeit pro Fließspur nicht mehr als eine Indikatorzone durchströmt, oder zueinander **nicht auf Lücke** angeordnet, so dass die Probenflüssigkeit pro Fließspur mehr als eine Indikatorzone durchströmt.
Mehr als eine solche Reihe von Indikatorzonen, beispielsweise zwei Reihen von Indikatorzonen, mit unterschiedlicher Distanz zur Aufgabezone sind insbesondere dann vorteilhaft, wenn aus einer Vollblutprobe zelluläre und plasmatische Parameter bestimmt werden sollen. In einer Ausführungsform, beispielhaft in einem Testansatz mit Vollblut als Probe, werden die Bindungselemente so gewählt, dass die im Plasma enthaltenen Analyten, zum Beispiel jede Art von Antikörpern, die durch die Vorrichtung von der Aufgabezone zum Absorptionsbereich durch die Indikatorzonen fließen, in der proximal zur Aufgabezone angeordneten Reihe von Indikatorzonen an die Bindungselemente binden. Die Bindungselemente zum Nachweis der zellulär gebundenen Analyten, beispielsweise erythrozytäre Antigene, werden dagegen so gewählt, dass diese in der distal zur Aufgabezone angeordneten Reihe von Indikatorzonen an die Bindungselemente binden. Bei dieser bevorzugten Ausführungsform wird vorzugsweise mit einer zusätzlich zu den Erythrozyten weiteren Sorte von Indikatorpartikeln, vorzugsweise aus kolloidalem Gold oder Polystyrol oder fixierten Erythrozyten, gearbeitet. Diese Indikatorpartikel werden insbesondere eingesetzt, um nicht erythrozytär gebundene Analyten, beispielsweise frei im Plasma vorkommende Antikörper, im Bindungskomplex in einer Indikatorzone sichtbar zu machen. Werden beispielhaft zwei Arten von Indikatorpartikeln verwendet, wovon eine nicht Erythrozyten sind, können die Indikatorzonen der beiden Reihen zueinander **nicht auf Lücke** bzw. in einer Fließspur hintereinander angeordnet sein. Vorteilhaft ist hierbei die Anordnung, bei der die aus dem Plasma nachgewiesenen Analyten in den proximalen Indikatorzonen nachgewiesen werden und bei der die Erythrozyten-gebundenen Analyten in den distalen Indikatorzonen nachgewiesen werden. Eine Ausführungsform der Erfindung mit mehr als einer, wie vorbeschriebenen Reihe von Indikatorzonen, von denen die Bindungselemente jeder Reihe mit Erythrozyten als Indikatorpartikel reagieren bzw. binden, sind die Reihen von Indikatorzonen zueinander **auf Lücke** bzw. in einer Fließspur nicht hintereinander angeordnet.

In einer weiteren und besonders bevorzugten **Ausführungsform** der Erfindung sind **mehr als eine solche Reihe** von Indikatorzonen, vorzugsweise in einer von proximal nach distal oder umgekehrt diagonal verlaufenden Reihe, oder beispielsweise in einer V-förmig, W-, M-, oder N-förmig oder umgekehrt V-förmig, W-, M-, oder N-förmig verlaufenden Reihe, oder beispielsweise auch in einer parallel nebeneinander versetzt verlaufenden Reihe, bidirektional (z. B. zu einem 180° Winkel) zu einer zentralen Aufgabezone angeordnet. Eine solche Anordnung ist insbesondere dann vorteilhaft, wenn aus einer Vollblutprobe zelluläre und plasmatische Parameter bestimmt werden sollen.
In einer Ausführungsform, beispielhaft in einem Testansatz mit Vollblut als Probe, werden die Bindungselemente so gewählt, dass die im Plasma enthaltenen Analyten, zum Beispiel jede Art von Antikörpern, die durch die Vorrichtung von der Aufgabezone zum Absorptionsbereich durch die Indikatorzonen fließen, in der auf der einen Seite zur Aufgabezone angeordneten Reihe von Indikatorzonen an die Bindungselemente binden. Die Bindungselemente zum Nachweis der zellulär gebundenen Analyten, beispielsweise erythrozytäre Antigene, werden dagegen so gewählt, dass diese in der auf der gegenüberliegenden Seite zur Aufgabezone angeordneten Reihe von Indikatorzonen an die Bindungselemente binden. Bei dieser bevorzugten Ausführungsform wird vorzugsweise mit einer zusätzlich zu den Erythrozyten weiteren Sorte von Indikatorpartikeln, vorzugsweise aus kolloidalem Gold oder Polystyrol, gearbeitet. Diese Indikatorpartikel werden insbesondere eingesetzt, um nicht erythrozytär gebundene Analyten, beispielsweise frei im Plasma vorkommende Antikörper, im Bindungskomplex in einer Indikatorzone sichtbar zu machen.

In einer bevorzugten Ausführungsform umfasst die eine Aufgabezone ferner zwei verschiedene Membranen unterschiedlicher Porosität. Bei dieser bevorzugten Ausführungsform wird vorzugsweise mit einer zusätzlich zu den Erythrozyten weiteren Sorte von Indikatorpartikeln, vorzugsweise aus kolloidalem Gold oder Polystyrol, gearbeitet. Diese Indikatorpartikel werden insbesondere eingesetzt, um nicht erythrozytär gebundene Analyten, beispielsweise frei im Plasma vorkommende Antikörper, im Bindungskomplex in einer Indikatorzone sichtbar zu machen.

In einer besonders bevorzugten Ausführungsform umfasst die eine Aufgabezone eine Membran oder zwei verschiedene Membranen unterschiedlicher Porosität. Ferner umfasst eine der beiden Membranen ein Konjugat-Pad, welches zwischen Dichtelement und Indikatorzonen angeordnet ist. Bei dieser bevorzugten Ausführungsform wird vorzugsweise mit einer zusätzlich zu den Erythrozyten weiteren Sorte von Indikatorpartikeln, vorzugsweise aus kolloidalem Gold oder Polystyrol, gearbeitet. Diese Indikatorpartikel werden insbesondere eingesetzt, um nicht erythrozytär gebundene Analyten, beispielsweise frei im Plasma vorkommende Antikörper, im Bindungskomplex in einer Indikatorzone sichtbar zu machen. Diese weitere zusätzlich zu den Erythrozyten eingesetzte Sorte von Indikatorpartikeln liegt vorzugsweise in das Konjugat-Pad eingetrocknet vor. Weiterhin sorgt das Konjugat-Pad dafür, dass der Fluss der Erythrozyten verlangsamt wird. Dies führt dazu, dass in der bidirektionalen Anordnung bei einer einzigen gemeinsamen Aufgabezone in der einen Richtung optimierte Bedingungen für den Nachweis zellulärer Eigenschaften und in der anderen Richtung optimierte Bedingungen für den Nachweis plasmatischer Eigenschaften bereitgestellt werden.

Durch die erfindungsgemäße Vorrichtung wird ein Lateral-Fluss-Test, insbesondere für die blutgruppenserologische Diagnostik, bereitgestellt, mit dem Erythrozyten als Indikatorpartikel verwendet werden und in einem Testansatz gleichzeitig mehrere zelluläre Parameter, insbesondere erythrozytäre Antigene bzw. Antigen-Epitope, plasmatische Parameter und/oder Blutzelleigenschaften, insbesondere aus Vollblutbestandteilen, pro zu untersuchender Probe bestimmt werden können. Des weiteren wird damit ein möglichst einfach herzustellendes und einfach, insbesondere mit wenigen Versuchsreihen und ohne Probenvorbereitung, zu handhabendes und kostengünstiges Testsystem bereitgestellt, mit dem gleichzeitig verschiedene zelluläre Parameter und/oder plasmatische Parameter einer Probe oder mehrerer zu untersuchender Proben bestimmt werden können.

Diese Vorteile bietet die erfindungsgemäße Vorrichtung auf jedem medizinischdiagnostischen Gebiet, bei dem gleichzeitig verschiedene zelluläre Parameter und plasmatische Parameter bestimmt werden sollen, insbesondere auch auf dem Gebiet der Blutgruppen- und Infektionsserologie, insbesondere für jegliche Diagnostik im Rahmen der Transfusionsmedizin, z. B. zur simultanen Durchführung von Blutgruppenbestimmung, wobei insbesondere Erythrozyten-gebundene Antigene bzw. Antigen-Epitope bestimmt werden, und Serumgegenprobe, wobei insbesondere reguläre Antikörper (Isoagglutinine) bestimmt werden, und/oder Antikörpersuch-Test, wobei insbesondere irreguläre Antikörper bestimmt werden, sowie zur simultanen Durchführung von Blutgruppenbestimmung und Bestimmung transfusionsrelevanter infektionsserologischer Marker, beispielhaft Antikörper gegen HIV-1, HIV-2, HCV, *Treponema pallidum,* sowie das Oberflächen-Antigen des Hepatitis B Virus (HbsAg), sowie zur simultanen Durchführung von Blutgruppenbestimmung und Bestimmung von Antikörpern gegen andere Blutzellen als Erythrozyten, insbesondere anti-thrombozytäre und anti-lymphozytäre Antikörper.

Hierzu kann antikoaguliertes oder natives Vollblut verwendet werden, bei dem vor der Bestimmung nicht in aufwendiger Weise Erythrozyten und Serum- bzw. Plasma-Fraktion von einander separiert werden müssen. Die Bestimmung kann in einem manuellen Format erfolgen, das komplett ohne Geräte (inklusive elektrischen Strom) auskommt.

In einer bevorzugten Ausführungsform der erfindungsgemäßen Vorrichtung **umfassen** die **Indikatorzonen,** vorzugsweise in einer distal zur Aufgabezone angeordneten Reihe von Indikatorzonen, **Antikörper bzw. Antikörperfragmente bzw. Lektine,** die die zu bestimmenden Blutgruppenantigene aller denkbaren Blutgruppensysteme und damit die sie tragenden Zellen in der Probe abfangen bzw. binden. Als bevorzugte Bindungselemente werden Antikörper bzw. Antikörperfragmente bzw. Lektine gegen Antigene oder Antigen-Epitope, insbesondere der Blutgruppensysteme AB0, Rh und Kell, beispielhaft anti-A, anti-B, anti-D und anti-K, in den Indikatorzonen an der porösen Membran, vorzugsweise in einer distal zu anderen Indikatorzonenreihen angeordneten Reihe, angebracht.
Vorzugsweise wird in einer Indikatorzone dieser Reihe von Indikatorzonen, vorzugsweise in einer distal zu allen übrigen Indikatorzonen dieser Reihe gelegenen Indikatorzone, ein Kontroll-Bindungselement (Kontrolle = ctl) angebracht, das den Durchfluss der Probe durch die Indikatorzonen positiv anzeigt. Das Kontroll-Bindungselement ist vorzugsweise ein polyklonaler anti-Erythrozyten-Antikörper.

Diese bevorzugte Ausführungsform der erfindungsgemäßen Vorrichtung umfasst in einer weiteren, vorzugsweise proximal zur Aufgabezone angeordneten Reihe von Indikatorzonen **Antigene bzw. Antigen-Epitope, die reguläre Antikörper** in der Probe abfangen bzw. binden. Als bevorzugte Bindungselemente werden dafür A1, A2, B, 0 Blutgruppenantigene bzw. -antigen-Epitope, beispielsweise Erythrozytenmembranen von Erythrozyten definierter Blutgruppen (A1, A2, B, 0) oder synthetisch hergestellte Blutgruppensubstanzen, an der porösen Membran angebracht. Vorzugsweise wird in einer Indikatorzone dieser Reihe von Indikatorzonen, vorzugsweise in einer distal zu allen übrigen Indikatorzonen dieser Reihe gelegenen Indikatorzone, ein Kontroll-Bindungselement (Kontrolle = ctl) angebracht, das den Durchfluss der Probe durch die Indikatorzonen positiv anzeigt. Das Kontroll-Bindungselement ist vorzugsweise ein anti-IgG Antikörper.

Diese bevorzugte Ausführungsform der erfindungsgemäßen Vorrichtung kann in einer weiteren, vorzugsweise proximal zur Aufgabezone angeordneten Reihe von Indikatorzonen **Antigene bzw. Antigen-Epitope** umfassen, **die irreguläre Antikörper** bzw. Fragmente davon in der Probe abfangen bzw. binden. Als bevorzugte Bindungselemente werden dafür die Zellmembranen verschiedener Blutgruppe 0 Erythrozyten-Präperationen, deren kombiniertes **Antigenprofil** diejenigen Antigene abdeckt, die gegen die wichtigsten Transfusions-relevanten irregulären Antikörper gerichtet sind, an der porösen Membran angebracht. Vorzugsweise wird in einer Indikatorzone dieser Reihe von Indikatorzonen, vorzugsweise in einer distal zu allen übrigen Indikatorzonen dieser Reihe gelegenen Indikatorzone, ein Kontroll-Bindungselement (Kontrolle = ctl) angebracht, das den Durchfluss der Probe durch die Indikatorzonen positiv anzeigt. Das Kontroll-Bindungselement ist vorzugsweise ein anti-IgG Antikörper.

In einer weiteren bevorzugten Ausführungsform der erfindungsgemäßen Vorrichtung **umfassen** die **Indikatorzonen,** vorzugsweise in einer distal zur Aufgabezone angeordneten Reihe von Indikatorzonen, **Antikörper bzw. Antikörperfragmente bzw. Lektine,** die die bei der Blutgruppenbestimmung zu bestimmenden Blutgruppenantigene und damit die sie tragenden Zellen in der Probe abfangen bzw. binden. Als bevorzugte Bindungselemente werden Antikörper bzw. Antikörperfragmente bzw. Lektine gegen Antigene oder Antigen-Epitope des AB0-Blutgruppensystems, beispielhaft anti-A, anti-B, anti-A und anti-B, in den Indikatorzonen an der porösen Membran, vorzugsweise in einer distal zur Aufgabezone und zu anderen Indikatorzonenreihen angeordneten Reihe, angebracht.
Vorzugsweise wird in einer Indikatorzone dieser Reihe von Indikatorzonen, vorzugsweise in einer distal zu allen übrigen Indikatorzonen dieser Reihe gelegenen Indikatorzone, ein Kontroll-Bindungselement (Kontrolle = ctl) angebracht, das den Durchfluss der Probe durch die Indikatorzonen positiv anzeigt. Das Kontroll-Bindungselement ist vorzugsweise ein polyklonaler anti-Erythrozyten-Antikörper.

Diese bevorzugte Ausführungsform der erfindungsgemäßen Vorrichtung umfasst in einer weiteren, vorzugsweise proximal zur Aufgabezone angeordneten Reihe von Indikatorzonen Thrombozyten- und/oder Lymphozytenmembranen bzw. Membranbestandteile als Bindungselemente zum Nachweis von antithrombozytären/lymphozytären Antikörpern.

In einer weiteren bevorzugten Ausführungsform der erfindungsgemäßen Vorrichtung **umfassen** die **Indikatorzonen,** vorzugsweise in einer distal zur Aufgabezone angeordneten Reihe von Indikatorzonen, **Antikörper bzw. Antikörperfragmente bzw. Lektine,** die die bei der Blutgruppenbestimmung zu bestimmenden Blutgruppenantigene und damit die sie tragenden Zellen in der Probe abfangen bzw. binden. Als bevorzugte Bindungselemente werden Antikörper bzw. Antikörperfragmente bzw. Lektine gegen Antigene oder Antigen-Epitope des AB0-Blutgruppensystems, beispielhaft anti-A, anti-B, anti-A und anti-B, in den Indikatorzonen an der porösen Membran, vorzugsweise in einer distal zur Aufgabezone und zu anderen Indikatorzonenreihen angeordneten Reihe, angebracht.

Vorzugsweise wird in einer Indikatorzone dieser Reihe von Indikatorzonen, vorzugsweise in einer distal zu allen übrigen Indikatorzonen dieser Reihe gelegenen Indikatorzone, ein Kontroll-Bindungselement (Kontrolle = ctl) angebracht, das den Durchfluss der Probe durch die Indikatorzonen positiv anzeigt. Das Kontroll-Bindungselement ist vorzugsweise ein polyklonaler anti-Erythrozyten-Antikörper.

Diese bevorzugte Ausführungsform der erfindungsgemäßen Vorrichtung umfasst in einer weiteren, vorzugsweise proximal zur Aufgabezone angeordneten Reihe von Indikatorzonen Bindungselemente zum Nachweis von infektiösen Agenzien, insbesondere synthetisch hergestellte Peptide oder mit rekombinanten DNA-Methoden exprimierte rekombinante Antigene, die diagnostisch bedeutsame Sequenzen von Oberflächenproteinen der jeweiligen Marker umfassen (Antikörper-Nachweis), oder Antikörper, welche gegen (Oberflächen-)Proteine infektiöser Agenzien gerichtet sind (Antigen-Nachweis).

Durch die erfindungsgemäße Vorrichtung muss für die gleichzeitige Bestimmung von zellulären und plasmatischen Parametern aus einer Probe nicht mehr für jede einzelne Bestimmung separat pipettiert werden, sondern an einer Probe können gleichzeitig sämtliche gewünschte Parameter bestimmt werden, insbesondere bei der simultanen Durchführung von Blutgruppenbestimmung und Serumgegenprobe und/oder Antikörpersuch-Test sowie bei der simultanen Durchführung von Blutgruppenbestimmung und Bestimmung transfusionsrelevanter infektionsserologischer Marker, wobei die Blutgruppenbestimmung mit dem Nachweis jeglicher infektionsserologischer Marker kombiniert werden kann, sowie bei der simultanen Durchführung von Blutgruppenbestimmung und Bestimmung von Antikörpern gegen andere Blutzellen als Erythrozyten, insbesondere anti-Thrombozyten und anti-Lymphozyten Antikörper.

Dies stellt eine außerordentliche Rationalisierung der Arbeitsabläufe dar. Neben dem Vorteil der Simultanbestimmung vieler serologischer Parameter ist hier der praktisch vollständige Wegfall der Probenvorbereitung im Vergleich zu herkömmlichen Tests zu nennen. Auch die Ablesung der in diagonaler Anordnung dargestellten Ergebnisse ist wesentlich günstiger. So lassen sich in der erfindungsgemäßen Vorrichtung beispielsweise Blutgruppen-, insbesondere AB0-Eigenschaften und Serumgegenprobe und Antikörpersuch-Test, oder Blutgruppen-, insbesondere AB0-Eigenschaften und andere immunhämatologische Parameter, insbesondere anti-thrombozytäre und/oder anti-lymphozytäre Antikörper bzw. Fragmente davon, oder Blutgruppen-, insbesondere AB0-Eigenschaften und infektionsserologische Marker, insbesondere Antikörper gegen bakterielle und/oder virale Agenzien bzw. Fragmente davon oder virale oder bakterielle Antigene bzw. -Epitope in einer Vorrichtung nebeneinander bestimmen und ablesen. Das zweidimensionale, flächige Ergebnis sowie der stabile Endpunkt der Reaktion begünstigen sowohl die Ablesung mit dem bloßen Auge als auch eine automatisierte Ablesung der Ergebnisse mit gängigen Bildanalyseverfahren, wie z. B. CCD-Kameras. Der Arbeitsaufwand ist vermindert, selbst bei manueller Abarbeitung. Die erfindungsgemäße Vorrichtung führt zudem zu einer Reduktion der Umweltbelastung und zu kostengünstigen Effekten. Selbst in Notsituationen mit Zeitdruck kann in kurzer Zeit in einer einzigen Versuchsanordnung beispielsweise eine komplette AB0-Blutgruppenbestimmung mit Serumgegenprobe und Antikörpersuch-Test nach irregulären Antikörpern durchgeführt werden, beispielsweise eine komplette AB0-Blutgruppenbestimmung mit infektionsserologischen Marker-Bestimmung oder anti-Thrombozyten/Lymphozyten Antikörper-Bestimmung durchgeführt werden.

Produktionstechnisch hat der Lateral-Diagonal-Fluss-Aufbau wesentliche Vorteile gegenüber dem Stand der Technik, indem es zu einem erheblich geringeren Verbrauch der verwendeten Reagenzien kommt und durch die Bereitstellung einer Vielzahl von Testparametern, welche bisher separat getestet werden müssen, in einer einzigen Vorrichtung.

Durch die erfindungsgemäße Vorrichtung wird ein Lateral-Fluss-Test, insbesondere für die immunhämatologische und infektionsserologische Diagnostik, bereitgestellt, mit dem in einem Testansatz gleichzeitig mehrere zelluläre, insbesondere erythrozytäre Antigene bzw. Antigen-Epitope, plasmatische Parameter und/oder Blutzelleigenschaften, insbesondere aus Vollblutbestandteilen, pro zu untersuchender Probe bestimmt werden können, wobei mindestens zwei Sorten von Indikatorpartikeln, von denen mindestens eine Sorte Erythrozyten sind, verwendet werden. Des weiteren wird damit ein möglichst einfach herzustellendes und einfach, insbesondere mit wenigen Versuchsreihen und ohne Probenvorbereitung, zu handhabendes und kostengünstiges Testsystem bereitgestellt, mit dem gleichzeitig verschiedene zelluläre Parameter und/oder plasmatische Parameter einer Probe, insbesondere Blutgruppenmerkmale, Nachweis regulärer und irregulärer Antikörper, Antikörper gegen Thrombozyten und/oder Lymphozyten und/oder infektionsserologischer Marker, insbesondere solcher mit transfusionsmedizinischer Relevanz, bestimmt werden können.

Die **Membran** der erfindungsgemäßen Vorrichtung ist eine poröse Membran. Bevorzugte Membran-Materialien sind beispielsweise Nitrozellulose (z. B. *UniSart* von Sartorius, *HiFlow* von Millipore, Whatman, *AE99* bzw. *FF85*/*100* von Schleicher & Schuell), Polyethylen (*Lateral Flo* von Porex Corporation) oder Nylon (*Novylon* von CUNO). Vorzugsweise weist die Membran eine möglichst große Porengröße auf, da eine hohe Porosität der Membran das Eindringen insbesondere von zellulären Komponenten der zu bestimmenden Probe, z. B. von Erythrozyten, in die poröse Struktur begünstigt. Von besonderem Vorteil ist der Einsatz aufnehmender Membranen. Die erfindungsgemäße Vorrichtung ist jedoch nicht auf diese Eigenschaften beschränkt. Bevorzugt werden alle Membranen mit einer hohen kapillaren Flussrate (Capillary Speed), wobei die kapillare Flussrate die Zeit ist, die eine Farblösung braucht, um 40 mm auf einer gegebenen Membran zurückzulegen. Besonders bevorzugt sind Membranen, deren kapillare Flussrate < 100 ist.

In der bidirektionalen Ausführungsform mit zwei unterschiedlichen Membranen hat die eine Membran vorzugsweise eine hohe kapillare Flussrate, vorzugsweise < 100, die andere Membran vorzugsweise eine geringere kapillare Flussrate, vorzugsweise > 100.

In einer bevorzugten Ausführungsform der Erfindung ist in Fließrichtung hinter der Aufgabezone der erfindungsgemäßen Vorrichtung auf der porösen Membran ein **Dichtelement** angeordnet. Zur Anwendung kommen zwei- oder dreidimensionale Dichtelemente, die auf der porösen Membran platziert werden und mit denen eine von der übrigen Fläche der porösen Membran separierte Probenauftragszone geschaffen wird. Das Dichtelement hat erfindungsgemäß primär die Wirkung einer Flüssigkeitsbarriere und erlaubt die gerichtete Verteilung von Probenflüssigkeit und Testreagenzien in die poröse Membran. Weiterhin dichtet das Dichtelement erfindungsgemäß die Probenauftragszone ab zur Verhinderung eines unerwünschten Flüssigkeitsübertritts in die anderen Bereiche der Lateral-Fluss-Vorrichtungsanordnung.

Bevorzugte Ausführungsformen des Dichtelementes sind die Steg- oder Trog- bzw. Trichter-Form. Die Ausformung des Dichtelementes erfolgt durch Schneideprozesse aus dem zur Herstellung des Dichtelementes verwendeten Material. Im Fall der Trichter- bzw. Trogform erhält das Dichtelement eine innere Öffnung, deren bevorzugte Ausführungsvarianten runde, quadratische oder rechteckige, im Fall der Trichterform sich zur Unterseite (Membrankontaktseite) des Dichtelementes verjüngende Formen sind.

Bevorzugte Materialien für das Dichtelement sind Materialien, die nicht wasseraufnehmend (hydrophob) sind. In einer besonderen Ausführungsform sind die Materialien einseitig mit einem Klebstofffilm, beispielsweise einem drucksensitiven bzw. selbsthaftenden Acrylatklebstoff, beschichtet. Somit kann das Dichtelement direkt auf die Oberfläche der porösen Membran geklebt werden. Alternativ kann das Dichtelement mit dem Lateral-Fluss-Gehäuse verbunden, beispielsweise verklebt sein, wobei in dieser Ausführungsform das Lateral-Fluss-Gehäuse das Dichtelement auf die Oberfläche der porösen Membran drückt und damit die Funktionen des Dichtelementes erzielt werden.

Bevorzugte Materialien für die Ausbildung von zweidimensionalen Dichtelementen sind jede Form von Klebebändern oder Klebefolien (z. B. Tesa 4124 von Beiersdorf AG, ARcare 7815 von Adhesives Research).

Bevorzugte Materialien für die Ausbildung von dreidimensionalen Dichtelementen sind flexible, geschlossenporige Elastomermaterialien oder flexible Silikonmaterialien mit unterschiedlichen Materialstärken, vorzugsweise 3-5 mm (z. B. Zellkautschuk EPDM140 von Pitzner, Silikonkautschuk oder Vollkautschuk, Härte 40° oder weniger, von Castan).

Durch diese erfindungsgemäße Ausgestaltung ist die erfindungsgemäße Vorrichtung in der Lage, flüssige Proben, die Zellen enthalten, wie beispielsweise Vollblut, aufzunehmen, ohne die Zellen dabei abzufiltern. Weiterhin erlaubt das Dichtelement das Auftragen großer Probenvolumina auf die poröse Membran (Aufgabezone), ohne dass diese überschwemmt wird. Somit unterstützt das Dichtelement die Nutzung der aufnehmenden Eigenschaften der porösen Membran. Weiter garantiert das Dichtelement einen gerichteten Probenfluss. Die erfindungsgemäße Vorrichtung kann jedoch mit oder ohne Dichtelement gut funktionieren.

Für den **Absorptionsbereich** (Absorptions-Pad) der erfindungsgemäßen Vorrichtung werden mechanisch stabile Materialien bevorzugt, vorzugsweise mit Wasserabsorptionskapazitäten von 20-30 g/100 cm² (z. B. Wicking Papier, Typ 300, Schleicher und Schüll). Der Kontakt zwischen dem Absorptions-Pad und der Lateral Fluss-Membran der erfindungsgemäßen Vorrichtung wird durch Andruck und Überlappung mit der porösen Membran hergestellt. Die genaue Positionierung des Absorptions-Pads auf der Membran wird durch Verkleben des Absorptions-Pads mit der, die Lateral Fluss-Membran tragenden Trägerschicht (backing sheet), erzielt.

Das Konjugat-Pad besteht vorzugsweise aus Glasfaser oder Cellulose und hat vorzugsweise die Eigenschaft, den Fluss nativer Erythrozyten zu verzögern.

In einer weiteren Ausführungsform sind die Komponenten der erfindungsgemäßen Vorrichtung zum Zwecke der mechanischen Verstärkung auf eine Unterlage bzw. **Trägerschicht** aufgebracht. Die erfindungsgemäße Vorrichtung kann jedoch mit oder ohne Trägerschicht funktionieren. Bevorzugt werden mechanisch stabile und nicht wasseraufnehmende Materialien, vorzugsweise mit Materialstärken von 100 µm oder mehr, die ein- oder zweiseitig mit einem Klebstofffilm, z. B. einem drucksensitiven bzw. selbsthaftenden Acrylatklebstoff, beschichtet sind (z.B. 0.005" Polyester W/ GL-187, G & L). Auf der Trägerschicht werden die poröse Membran und das Absorptions-Pad fixiert. Im Fall der doppelseitig klebenden Trägerschicht wird die klebende zweite Seite zur Fixierung des Stapels auf weiteren Flächen, z. B. innerhalb der Lateral-Fluss-Gehäuse, eingesetzt.

In einer weiteren Ausführungsform ist die erfindungsgemäße Vorrichtung, entweder mit oder ohne Trägerschicht, auf der die Komponenten der erfindungsgemäßen Vorrichtung aufgebracht sind, in einem **Gehäuse** integriert, wodurch die Membran-Komponenten aneinander gedrückt werden und das Gehäuse die Dichtelementfunktion unterstützt. Dabei kann die erfindungsgemäße Vorrichtung jedoch mit oder ohne Gehäuse genauso gut funktionieren.

Ein weiterer Gegenstand der Erfindung ist die **Verwendung** der erfindungsgemäßen Vorrichtung zur Analyse von Blut, insbesondere zur simultanen Durchführung der Blutgruppenbestimmung und Serumgegenprobe und/oder Antikörpersuch-Test und/oder zur simultanen Durchführung der Blutgruppenbestimmung und des Nachweises von Antikörpern gegen infektiöse, insbesondere bakterielle und/oder virale Agenzien bzw. Fragmenten davon oder von Antigenen infektiöser Agenzien und/oder zur simultanen Durchführung der Blutgruppenbestimmung und des Nachweises von Antikörpern gegen andere Blutzellen als Erythrozyten, insbesondere anti-thrombozytärer oder anti-lymphozytärer Antikörper, bzw. Fragmenten davon.

Die Aufgabe wird erfindungsgemäß gelöst zum anderen durch ein **Verfahren** zur Bestimmung mehrerer Analyten oder deren Derivaten in einer flüssigen Probe, welches das Auftragen der Probe auf die Aufgabezone einer Membran der erfindungsgemäßen Vorrichtung umfasst, wobei diese Probe in ausreichender Menge vorliegt, um die Probenflüssigkeit dazu zu veranlassen, in Richtung Absorptionsbereich durch die Indikatorzonen zu fließen und um die Analyten oder ihre Derivate in der Probenflüssigkeit dazu zu veranlassen, an die jeweiligen Indikatorzonen zu binden bzw. in den Indikatorzonen einen Komplex zu bilden.

Eine besondere Ausführungsform des erfindungsgemäßen Verfahrens führt simultan eine Blutgruppenbestimmung und Serumgegenprobe und/oder Antikörpersuch-Test durch.
Beispielhaft wird Vollblut oder eine Verdünnung davon auf die Aufgabezone der Vorrichtung aufgetragen. Alle Komponenten dringen, geführt durch das Dichtelement, in die poröse Membran ein und passieren bei der Migration in Richtung Absorptions-Pad zunächst den Indikatorzonenbereich Serumgegenprobe, der Fragmente der Zellen A1, A2, B, 0 umfassen sowie die Kontroll-Indikatorzone mit anti-IgG/IgM. Die im Serum vorhandenen Isoagglutinine binden an die korrespondierenden zellulär gebundenen Antigene. Serum-IgG bindet an das Kontrollbindungselement (Serumgegenprobe: Sensibilisierung).
Die zellulär gebundenen Antigene migrieren weiter in den distal zum Indikatorzonenbereich Serumgegenprobe gelegenen Indikatorzonenberecich Blutgruppenbestimmung, in dem in jeder Indikatorzone ein Antikörper gegen ein anderes Blutgruppenmerkmal immobilisiert ist (z. B. anti-A, anti-B, anti-AB). Die zur Aufgabezone distalste Indikatorzone dieses Bereiches hat beispielhaft polyklonale anti-Erythrozyten-Antikörper als Bindungselement. In diesem Indikatorzonenbereich binden die Erythrozyten an die Bindungselemente, die zu den jeweiligen Blutgruppenmerkmalen korrespondieren. An das Kontrollbindeelement binden Erythrozyten jedweder Blutgruppe (Blutgruppenbestimmung).
In einem folgenden Waschschritt wird ungebundenes Material aus der Membran ausgewaschen. Im nachfolgenden Detektionsschritt werden mit Hilfe von synthetischen Partikeln, welche mit anti-IgG/IgM beschichtet sind, die an den Bindungselementen der proximalen Reihe von Indikatorzonen immobiliserten Isoagglutinine und Kontrollantikörper sichtbar gemacht (Serumgegenprobe: Detektion).

In einer anderen Ausführungsform des erfindungsgemäßen Verfahrens zur simultanen Blutgruppenbestimmung und Serumgegenprobe und/oder Antikörpersuch-Test werden die Blutgruppenmerkmale wie oben beschrieben direkt bestimmt, hingegen Serumgegenprobe und/oder Antikörpersuchtest als Kompetitionstest durchgeführt:
Beispielhaft wird Vollblut oder eine Verdünnung davon auf die Aufgabezone der Vorrichtung aufgetragen. Alle Komponenten dringen, geführt durch das Dichtelement, in die poröse Membran ein und passieren bei der Migration in Richtung Absorptions-Pad zunächst den Indikatorzonenbereich Serumgegenprobe, der Fragmente von Zellen der Blutgruppen A und B umfasst sowie die Kontroll-Indikatorzone, die Fragmente von Zellen der Blutgruppe 0 umfasst. Die im Serum vorhandenen Isoagglutinine binden an die korrespondierenden zellulär gebundenen Antigene. (Serumgegenprobe: Sensibilisierung).
Die zellulär gebundenen Antigene migrieren weiter in den distal zum Indikatorzonenbereich Serumgegenprobe gelegenen Indikatorzonenbereich Blutgruppenbestimmung, in dem in jeder Indikatorzone ein Antikörper gegen ein anderes Blutgruppenmerkmal immobilisiert ist (z. B. anti-A, anti-B, anti-D). Die zur Aufgabezone distalste Indikatorzone dieses Bereiches hat beispielhaft polyklonale anti-Erythrozyten-Antikörper als Bindungselement. In diesem Indikatorzonenbereich binden die Erythrozyten an die Bindungselemente, die zu den jeweiligen Blutgruppenmerkmalen korrespondieren. An das Kontrollbindeelement binden Erythrozyten jedweder Blutgruppe (Blutgruppenbestimmung).
In einem folgenden Waschschritt wird ungebundenes Material aus der Membran ausgewaschen. Im nachfolgenden Schritt wird eine Suspension von unterschiedlichen synthetischen Partikeln, welche jeweils mit anti-A, anti-B oder anti-H beschichtet sind, auf die Aufgabezone aufgetragen. Die Partikel können jeweils nur an diejenigen Bindungselemente im Indikatorzonenbereich Serumgegenprobe binden, die im Sensibilisierungsschritt nicht mit den Serum-Isoagglutininen in Kontakt gekommen waren, d. h. eine farbige Bande zeigt in diesem Falle die Abwesenheit des entsprechenden Isoagglutinins an. Beispielhaft werden im Falle einer Blutgruppe A (Personen mit Isoagglutininen anti-B) die Zellfragmente der B-Zellen durch die Isoagglutinine blockiert, was dazu führt, dass die Zellfragmente der A-Zellen durch das nachfolgend aufgetragene Gemisch synthetischer Partikel durch die darin vorhandenen anti-A Partikel angefärbt werden. Die Blutgruppe 0 Fragmente werden in allen denkbaren Konstellationen angefärbt, da sie nicht durch Isoagglutinine blockiert werden und dadurch immer frei sind für eine Reaktion mit den gefärbten anti-H Partikeln, wodurch auch in der Variante des Kompetitionsassays ein Kontrollelement bereitgestellt wird (Serumgegenprobe: Detektion).

In einer weiteren Ausführungsform des erfindungsgemäßen Verfahrens zur simultanen Blutgruppenbestimmung und Serumgegenprobe und/oder Antikörpersuch-Test mit direkter Bestimmung der Blutgruppenmerkmale und Bestimmung der Serumgegenprobe und/oder Antikörpersuchtest als Kompetitionstest wird wie folgt vorgegangen:
Vollblut oder eine Verdünnung davon wird auf die asymmetrische Aufgabezone der Vorrichtung mit zwei unterschiedlichen porösen Membranenaufgetragen. Eine poröse Membran weist dabei eine geringere kapillare Flussrate als die andere Membran auf. Bei letzterer ist zwischen dem Dichtelement und den Indikatorzonen ein Konjugat-Pad, das aufgetrocknete anti-A/anti-B/anti-H-Partikel enthält, auf der Membran aufgebracht. Alle Komponenten dringen, geführt durch das Dichtelement, in beide poröse Membranen ein, was folgende Flussverhalten bewirkt:
"Nur-Membran-Seite": Alle Komponenten passieren bei der Migration in Richtung des dazugehörenden Absorptions-Pads den Indikatorzonenbereich Blutgruppenbestimmung, in dem in jeder Indikatorzone ein Antikörper gegen ein anderes Blutgruppenmerkmal immobilisiert ist (z. B. anti-A, anti-B, anti-D). Die zur Aufgabezone distalste Indikatorzone dieses Bereiches hat beispielhaft polyklonale anti-Erythrozyten-Antikörper als Bindungselement. In diesem Indikatorzonenbereich binden die Erythrozyten an die Bindungselemente, die zu den jeweiligen Blutgruppenmerkmalen korrespondieren. An das Kontrollbindeelement binden Erythrozyten jedweder Blutgruppe.
"Membran/Konjugat-Pad-Seite": Alle Komponenten kommen nach Eindringen in die Membran und Passieren des Dichtelements mit dem Konjugat-Pad in Kontakt, wobei die zellulären Bestandteile festgehalten bzw. gebremst werden und die flüssigen Komponenten (Plasma) ungehindert weiterfliessen können. Letztere lösen die anti-A/anti-B/anti-H Partikel aus dem Konjugat-Pad. Der Indikatorzonenbereich Serumgegenprobe umfasst Fragmente von Zellen der Blutgruppen A und B sowie als Kontrollelement Fragmente von Zellen der Blutgruppe 0. Die im Serum vorhandenen Isoagglutinine binden an die korrespondierenden zellulär gebundenen Antigene und kompetieren damit um eine Bindung der farbigen anti-A, anti-B Partikel. Das bedeutet, dass die Partikel nur binden können, wenn das jeweilige Isoagglutinin nicht vorhanden ist. Beispielsweise hat eine Blutgruppe A Person anti-B Isoagglutinine, was dazu führt, dass die Blutgruppe-B Fragmente im Indikatorzonenbereich blockiert werden und nur die Blutgruppe A Fragmente durch die farbigen anti-A Partikel angefärbt werden können. Das Kontrollelement, gegen das keine Isoagglutinine existieren, bleibt damit immer unblockiert und wird durch die in der Partikelmischung des Konjugat-Pads enthaltenen anti-H Partikel als sichtbare Bande angefärbt.

Eine weitere besondere Ausführungsform des erfindungsgemäßen Verfahrens führt simultan eine Blutgruppenbestimmung und eine Bestimmung von anti-Thrombozyten und/oder anti-Lymphozyten Antikörper bzw. Fragmenten davon durch.
Beispielhaft umfassen für die Bestimmung der anti-thrombozytären Antikörper die Indikatorzonen im zur Aufgabezone proximalen Bereich Thrombozytenfragmente als Bindungselemente, an die -falls in der Probe vorhanden- im Serum enthaltene anti-thrombozytäre Antikörper binden. Das übrige Verfahren ist wie in der vorstehenden Ausführungsform des erfindungsgemäßen Verfahrens beschrieben, insbesondere erfolgt die Blutgruppenbestimmung wie dort beschrieben.
Eine weitere besondere Ausführungsform des erfindungsgemäßen Verfahrens führt simultan eine Blutgruppenbestimmung und eine Bestimmung transfusionsrelevanter infektionsserologischer Marker durch.
Beispielhaft umfassen für die Bestimmung von Infektionsmarkern die Indikatorzonen des ersten, proximalen Indikatorzonenbereichs als Bindungselemente synthetische Peptide und/oder rekombinante Proteine, die den Sequenzen von Proteinen viraler oder bakterieller Agenzien entsprechen, (Bestimmung von Antikörpern gegen Infektionsmarker, z. B. anti-HIV-1), sowie Antikörper, die gegen Proteine von Infektionsmarkern gerichtet sind (Bestimmung von Antigenen, z. B. HbsAg). Im Serum enthaltene Antikörper bzw. Antigene binden im ersten Schritt an die korrespondierenden Antigene bzw. Antikörper. Das übrige Verfahren ist wie in der vorstehenden Ausführungsform des erfindungsgemäßen Verfahrens beschrieben, insbesondere erfolgt die Blutgruppenbestimmung wie dort beschrieben.

Bei dem erfindungsgemäßen Verfahren handelt es sich bei den zu bestimmenden **Analyten** insbesondere um Blutgruppen-Antigene bzw. Antigen-Epitope, vorzugsweise solche der Blutgruppensysteme AB0, Rh, Kell, um gegen Blutgruppenantigene oder -antigen-Epitope gerichtete Antikörper bzw. Fragmente davon, vorzugsweise reguläre Antikörper, irreguläre Antikörper, um Antikörper gegen infektiöse Agenzien, um (Oberflächen-)Antigene infektiöser Agenzien und/oder um anti-thrombozytäre oder anti-lymphozytäre Antikörper bzw. Fragmente davon.

Die zu untersuchende Probe, beispielsweise natives oder antikoaguliertes Vollblut oder Erythrozytenkonzentrate oder verdünnte Erythrozyten-Suspensionen, Blutbestandteile oder Testflüssigkeiten, wie Kontrollserum oder Kontrollzellen, wird auf die Aufgabezone der erfindungsgemäßen Vorrichtung aufgetragen. Die in der Probe enthaltenen Erythrozyten, die den/die Analyten tragen, dienen gleichzeitig als Indikatorpartikel.

Es werden insbesondere zwei Gruppen von Indikatorpartikeln verwendet, von denen mindestens eine Sorte Erythrozyten sind. Die eine wird alleine durch Erythrozyten zum direkten Nachweis von Erythrozyten-gebundenen Analyten repräsentiert. Die andere Gruppe besteht aus Partikeln jeder denkbaren Art und Kombination, mit denen sich Bindungsreaktionen nachweisen lassen, vorzugsweise Partikel aus kolloidalem Gold oder aus Polystyrol oder fixierte Erythrozyten.

Im folgenden wird die Erfindung durch Figuren und Beispiele näher erläutert, ohne sie einzuschränken. Es zeigen:
- Fig. 1: eine perspektivische Darstellung einer erfindungsgemäßen Vorrichtung für Lateral-Fluss-Tests für die simultane Durchführung von Blutgruppenbestimmung und Serumgegenprobe;
- Fig. 2: eine Explosionsdarstellung der in Fig. 1 dargestellten erfindungsgemäßen Vorrichtung für Lateral-Fluss-Tests;
- Fig. 3: eine perspektivische Darstellung einer erfindungsgemäßen Vorrichtung für Lateral-Fluss-Tests für die simultane Durchführung von Blutgruppenbestimmung und Serumgegenprobe, ausgeführt mit einem dreidimensionalen Dichtelement in Stegform;
- Fig. 4: eine Explosionsdarstellung der in Fig. 3 dargestellten erfindungsgemäßen Vorrichtung für Lateral-Fluss-Tests;
- Fig. 5: eine perspektivische Darstellung einer erfindungsgemäßen Vorrichtung für Lateral-Fluss-Tests für die simultane Durchführung von Blutgruppenbestimmung und Serumgegenprobe, ausgeführt mit einem dreidimensionalen Dichtelement in Trogform;
- Fig. 6: eine Explosionsdarstellung der in Fig. 5 dargestellten erfindungsgemäßen Vorrichtung für Lateral-Fluss-Tests;
- Fig. 7: eine perspektivische Darstellung einer erfindungsgemäßen Vorrichtung für Lateral-Fluss-Tests für die simultane Durchführung von Blutgruppenbestimmung, Serumgegenprobe und Antikörpersuchtest für Empfänger;
- Fig. 8: eine perspektivische Darstellung einer erfindungsgemäßen Vorrichtung für Lateral-Fluss-Tests für die simultane Durchführung von Blutgruppenbestimmung, Serumgegenprobe und Antikörpersuchtest bei Spendern;
- Fig. 9: eine perspektivische Darstellung einer erfindungsgemäßen Vorrichtung für Lateral-Fluss-Tests für die simultane Durchführung von Blutgruppenbestimmung und dem Nachweis von Infektionsmarkern;
- Fig. 10: eine perspektivische Darstellung einer erfindungsgemäßen Vorrichtung für Lateral-Fluss-Tests für die simultane Durchführung von Blutgruppenbestimmung und dem Nachweis von Antikörpern gegen thrombozytäre Antigene.
- Fig. 11: zeigt eine perspektivische Darstellung einer erfindungsgemäßen Vorrichtung für Lateral-Fluss-Tests mit bidirektionalem Fluss für die simultane Bestimmung von Blutgruppenbestimmung und Serumgegenprobe;
- Fig. 12: zeigt eine Explosionsdarstellung der in Fig. 17 dargestellten erfindungsgemäßen Vorrichtung für Lateral-Fluss-Tests.

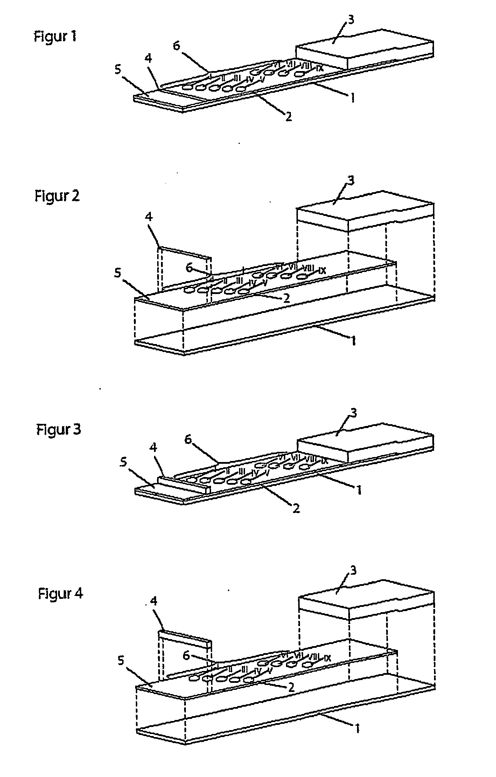

In Fig. 1 wird beispielhaft eine perspektivische Darstellung einer erfindungsgemäßen Vorrichtung für Lateral-Fluss-Tests für die simultane Durchführung von Blutgruppenbestimmung und Serumgegenprobe gezeigt. Im vorliegenden Beispiel besteht die Vorrichtung aus einer Trägerschicht 1, der porösen Membran 2, dem Absorptions-Pad 3 und dem zweidimensionalen, in Stegform ausgeführten Dichtelement 4. Dabei ist die poröse Membran 2 auf der mit einem drucksensitiven Acrylatklebstoff versehenen Trägerschicht 1 fixiert. Ebenso ist das Absorptions-Pad 3 auf der Trägerschicht 1 fixiert, wobei ein Teil des Absorptions-Pad 3 mit der porösen Membran 2 überlappt. Das auf der Oberseite der porösen Membran 2 fixierte Dichtelement 4 separiert die Aufgabezone 5 von der übrigen Membranfläche und ermöglicht die gerichtete Verteilung von Probenflüssigkeit und Testreagenzien in die poröse Membran 2. Zwischen der Aufgabezone 5 und dem Bereich der porösen Membran 2, der mit dem Absorptions-Pad 3 in Kontakt steht, ist der Indikatorzonenbereich 6, angeordnet. Dieser wird aus diagonal versetzten, in definierten X- und Y-Positionen angeordneten, punktförmigen Indikatorzonen I-IX gebildet, wobei die Indikatorzonen I-V den Indikatorzonenbereich "Serumgegenprobe" und die Indikatorzonen VI-IX den Indikatorzonenbereich "Blutgruppenbestimmung" umfassen und aus den folgenden Bindungselementen bestehen:

| Indikatorzone | Bindungselement | Spezifikation |
|---|---|---|
| | | |
| *Indikatorzonenbereich: Serumgegenprobe* | | |
| | | |
| I | Erythrozyten-Ghosts | Blutgruppe A1 |
| II | Erythrozyten-Ghosts | Blutgruppe A2 |
| III | Erythrozyten-Ghosts | Blutgruppe B |
| IV | Erythrozyten-Ghosts | Blutgruppe 0 |
| V | Antikörper | Anti-human IgG/IgM |
| | | |
| *Indikatorzonenbereich:Blutgruppenbestimmung* | | |
| | | |
| VI | Antikörper | Anti-A (monoklonal) |
| VII | Antikörper | Anti-B (monoklonal) |
| VIII | Antikörper | Anti-AB (monoklonal) |
| IX | Antikörper | Anti-Erythrozyten (polyklonal) |

Indikatorzone V ist die Kontrolle (ctl) für die Serumgegenprobe und enthält anti-human IgG/IgM Antikörper. Indikatorzone IX ist die Kontrolle (ctl) für die Blutgruppenbestimmung und enthält polyklonale anti-Erythrozyten Antikörper. Sie befindet sich distal angeordnet zu allen übrigen Indikatorzonen.

In Fig. 2 wird eine Explosionsdarstellung der in Fig. 1 dargestellten erfindungsgemäßen Vorrichtung für Lateral-Fluss-Tests gezeigt, die aus den Komponenten Trägerschicht 1, poröse Membran 2, Absorptions-Pad 3 und Dichtelement 4 besteht, welches die Aufgabezone 5 von der übrigen Membran separiert, die wiederum den Indikatorzonenbereich 6, bestehend aus den Indikatorzonenbereichen "Serumgegenprobe" und "Blutgruppenbestimmung" mit den diagonal versetzt angeordneten Indikatorzonen I-IX enthält.

In Fig. 3 wird beispielhaft eine perspektivische Darstellung einer erfindungsgemäßen Vorrichtung für Lateral-Fluss-Tests für die simultane Durchführung von Blutgruppenbestimmung und Serumgegenprobe gezeigt. Im vorliegenden Beispiel entsprechen die Komponenten der Vorrichtung den Komponenten der in Fig. 1 dargestellten Vorrichtung mit Ausnahme des auf der Oberseite der porösen Membran 2 fixierten, in dreidimensionaler Stegform ausgeführten Dichtelements 4.

In Fig. 4 wird eine Explosionsdarstellung der in Fig. 3 dargestellten erfindungsgemäßen Vorrichtung für Lateral-Fluss-Tests mit den Komponenten Trägerschicht 1, poröse Membran 2, Absorptions-Pad 3 und in dreidimensionaler Stegform ausgeführtes Dichtelement 4 gezeigt, welches die Aufgabezone 5 von der übrigen Membran separiert, die wiederum den Indikatorzonenbereich 6, bestehend aus den Indikatorzonenbereichen "Serumgegenprobe" und "Blutgruppenbestimmung" mit den diagonal versetzt angeordneten Indikatorzonen I-IX enthält.

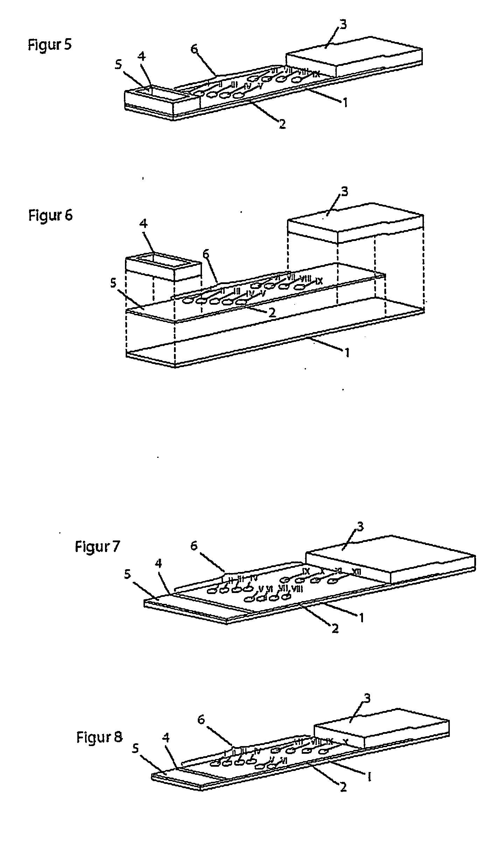

In Fig. 5 wird beispielhaft eine perspektivische Darstellung einer erfindungsgemäßen Vorrichtung für Lateral-Fluss-Tests für die simultane Durchführung von Blutgruppenbestimmung und Serumgegenprobe gezeigt. Im vorliegenden Beispiel entsprechen die Komponenten der Vorrichtung den Komponenten der in Fig. 1 dargestellten Vorrichtung mit Ausnahme des auf der Oberseite der porösen Membran 2 fixierten, in dreidimensionaler Trogform ausgeführten Dichtelements 4.

In Fig. 6 wird eine Explosionsdarstellung der in Fig. 5 dargestellten erfindungsgemäßen Vorrichtung für Lateral-Fluss-Tests mit den Komponenten Trägerschicht 1, poröse Membran 2, Absorptions-Pad 3 und in dreidimensionaler Trogform ausgeführtes Dichtelement 4 gezeigt, welches die Aufgabezone 5 von der übrigen Membran separiert, die wiederum den Indikatorzonenbereich 6, bestehend aus den Indikatorzonenbereichen "Serumgegenprobe" und "Blutgruppenbestimmung" mit den diagonal versetzt angeordneten Indikatorzonen I-IX enthält.

In Fig. 7 wird beispielhaft eine perspektivische Darstellung einer erfindungsgemäßen Vorrichtung für Lateral-Fluss-Tests für die simultane Durchführung von Blutgruppenbestimmung, Serumgegenprobe und Antikörpersuchtest bei Empfängern gezeigt. Im vorliegenden Beispiel besteht die Vorrichtung aus einer Trägerschicht 1, der porösen Membran 2, dem Absorptions-Pad 3 und dem zweidimensionalen, in Stegform ausgeführten Dichtelement 4. Dabei ist die poröse Membran 2 auf der mit einem drucksensitiven Acrylatklebstoff versehenen Trägerschicht 1 fixiert. Ebenso ist das Absorptions-Pad 3 auf der Trägerschicht 1 fixiert, wobei ein Teil des Absorptions-Pad 3 mit der porösen Membran 2 überlappt. Das auf der Oberseite der porösen Membran 2 fixierte Dichtelement 4 separiert die Aufgabezone 5 von der übrigen Membranfläche und ermöglicht die gerichtete Verteilung von Probenflüssigkeit und Testreagenzien in die poröse Membran 2. Zwischen der Aufgabezone 5 und dem Bereich der porösen Membran 2, der mit dem Absorptions-Pad 3 in Kontakt steht, ist der Indikatorzonenbereich 6 angeordnet. Dieser wird aus diagonal versetzten, in definierten X- und Y-Positionen angeordneten, punktförmigen Indikatorzonen I-XII gebildet, wobei die Indikatorzonen I-VIII den Indikatorzonenbereich "Serumgegenprobe/Antikörpersuchtest" und die Indikatorzonen IX-XII den Indikatorzonenbereich "Blutgruppenbestimmung" umfassen und aus den folgenden Bindungselementen bestehen:

| Indikatorzone | Bindungselement | Spezifikation |
|---|---|---|
| | | |
| *Indikatorzonenbereich: Serumgegenprobe*/*Antikörpersuche* | | |
| | | |
| I | Erythrozyten-Ghosts | Blutgruppe A1 |
| II | Erythrozyten-Ghosts | Blutgruppe A2 |
| III | Erythrozyten-Ghosts | Blutgruppe B |
| IV | Erythrozyten-Ghosts | Blutgruppe 0 |
| V | Erythrozyten-Ghosts | Suchzelle 1, Blutgruppe 0, Rh-Formel R₁R₁^{W} |
| VI | Erythrozyten-Ghosts | Suchzelle 2, Blutgruppe 0, Rh-Formel R₂R₂ |
| VII | Erythrozyten-Ghosts | Suchzelle 3, Blutgruppe 0, Rh-Formel R₁R₁ |
| VIII | Antikörper | Anti-human IgG/IgM |
| | | |
| *Indikatorzonenbereich: Blutgruppenbestimmung* | | |
| | | |
| IX | Antikörper | Anti-A (monoklonal) |
| X | Antikörper | Anti-B (monoklonal) |
| XI | Antikörper | Anti-AB (monoklonal) |
| XII | Antikörper | Anti-Erythrozyten (polyklonal) |

Indikatorzone VII ist die Kontrolle (ctl) für die Serumgegenprobe und Antikörpersuchtest und enthält anti-human IgG/IgM Antikörper. Indikatorzone XII ist die Kontrolle (ctl) für die Blutgruppenbestimmung und enthält polyklonale anti-Erythrozyten Antikörper. Sie befindet sich distal angeordnet zu allen übrigen Indikatorzonen.

In Fig. 8 wird beispielhaft eine perspektivische Darstellung einer erfindungsgemäßen Vorrichtung für Lateral-Fluss-Tests für die simultane Durchführung von Blutgruppenbestimmung, Serumgegenprobe und Antikörpersuchtest bei Blutspendern gezeigt. Im vorliegenden Beispiel besteht die Vorrichtung aus einer Trägerschicht 1, der porösen Membran 2, dem Absorptions-Pad 3 und dem zweidimensionalen, in Stegform ausgeführten Dichtelement 4. Dabei ist die poröse Membran 2 auf der mit einem drucksensitiven Acrylatklebstoff versehenen Trägerschicht 1 fixiert. Ebenso ist das Absorptions-Pad 3 auf der Trägerschicht 1 fixiert, wobei ein Teil des Absorptions-Pad 3 mit der porösen Membran 2 überlappt. Das auf der Oberseite der porösen Membran 2 fixierte Dichtelement 4 separiert die Aufgabezone 5 von der übrigen Membranfläche und ermöglicht die gerichtete Verteilung von Probenflüssigkeit und Testreagenzien in die poröse Membran 2. Zwischen der Aufgabezone 5 und dem Bereich der porösen Membran 2, der mit dem Absorptions-Pad 3 in Kontakt steht, ist der Indikatorzonenbereich 6 angeordnet. Dieser wird aus diagonal versetzten, in definierten X- und Y-Positionen angeordneten, punktförmigen Indikatorzonen I-X gebildet, wobei die Indikatorzonen I-VI den Indikatorzonenbereich "Serumgegenprobe/Antikörpersuchtest" und die Indikatorzonen VII-X den Indikatorzonenbereich "Blutgruppenbestimmung" umfassen und aus den folgenden Bindungselementen bestehen:

| Indikatorzone | Bindungselement | Spezifikation |
|---|---|---|
| | | |
| *Indikatorzonenbereich: SerumgegenprobelAntikörpersuche* | | |
| | | |
| I | Erythrozyten-Ghosts | Blutgruppe A1 |
| II | Erythrozyten-Ghosts | Blutgruppe A2 |
| III | Erythrozyten-Ghosts | Blutgruppe B |
| IV | Erythrozyten-Ghosts | Blutgruppe 0 |
| V | Erythrozyten-Ghosts | Blutgruppe 0, Pool der Suchzellen 1, 2, 3 (siehe Beschreibung Fig. 8) |
| VI | Antikörper | Anti-human IgG/IgM |
| | | |
| *Indikatorzonenbereich: Blutgruppenbestimmung* | | |
| | | |
| VII | Antikörper | Anti-A (monoklonal) |
| VIII | Antikörper | Anti-B (monoklonal) |
| IX | Antikörper | Anti-AB (monoklonal) |
| X | Antikörper | Anti-Erythrozyten (polyklonal) |

Indikatorzone VI ist die Kontrolle (ctl) für die Serumgegenprobe und Antikörpersuchtest und enthält anti-human IgG/IgM Antikörper. Indikatorzone X ist die Kontrolle (ctl) für die Blutgruppenbestimmung und enthält polyklonale anti-Erythrozyten Antikörper. Sie befindet sich distal angeordnet zu allen übrigen Indikatorzonen.

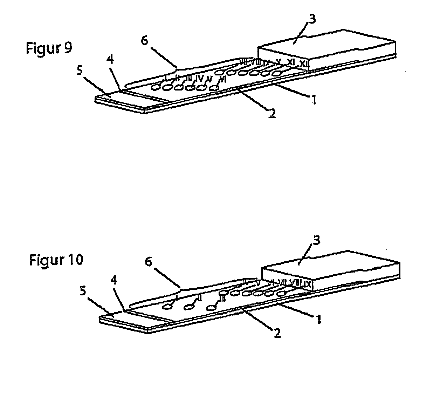

In Fig. 9 wird beispielhaft eine perspektivische Darstellung einer erfindungsgemäßen Vorrichtung für Lateral-Fluss-Tests für die simultane Durchführung von Blutgruppenbestimmung und Nachweis von Infektionsmarkern gezeigt. Im vorliegenden Beispiel besteht die Vorrichtung aus einer Trägerschicht 1, der porösen Membran 2, dem Absorptions-Pad 3 und dem zweidimensionalen, in Stegform ausgeführten Dichtelement 4. Dabei ist die poröse Membran 2 auf der mit einem drucksensitiven Acrylatklebstoff versehenen Trägerschicht 1 fixiert. Ebenso ist das Absorptions-Pad 3 auf der Trägerschicht 1 fixiert, wobei ein Teil des Absorptions-Pad 3 mit der porösen Membran 2 überlappt. Das auf der Oberseite der porösen Membran 2 fixierte Dichtelement 4 separiert die Aufgabezone 5 von der übrigen Membranfläche und ermöglicht die gerichtete Verteilung von Probenflüssigkeit und Testreagenzien in die poröse Membran 2. Zwischen der Aufgabezone 5 und dem Bereich der porösen Membran 2, der mit dem Absorptions-Pad 3 in Kontakt steht, ist der Indikatorzonenbereich 6 angeordnet. Dieser wird aus diagonal versetzten, in definierten X- und Y-Positionen angeordneten, punktförmigen Indikatorzonen I-XII gebildet, wobei die Indikatorzonen I-VI den Indikatorzonenbereich "Nachweis von Infektionsmarkern" und die Indikatorzonen VII-XII den Indikatorzonenbereich "Blutgruppenbestimmung" umfassen und aus den folgenden Bindungselementen bestehen:

| Indikatorzone | Bindungselement | Spezifikation |
|---|---|---|
| | | |
| *Indikatorzonenbereich: Nachweis von Infektionsmarkern* | | |
| | | |
| I | Synthetische Peptide | HIV-1 (gp-14, gp-41) |
| II | Synthetische Peptide | HIV-2 (gp-36) |
| III | Antikörper | Anti-HBsAg (monoklonal) |
| IV | Rekombinantes Antigen | HCV (C-100, C-200, C33c, C22) |
| V | Rekombinantes Antigen | Syphilis (TpN 15, TpN 17, TpN 47) |
| VI | Antikörper | Anti-human IgG/IgM |
| | | |
| *Indikatorzonenbereich: Blutgruppenbestimmung* | | |
| | | |
| VII | Antikörper | Anti-A (monoklonal) |
| VIII | Antikörper | Anti-B (monoklonal) |
| IX | Antikörper | Anti-AB (monoklonal) |
| X | Antikörper | Anti-D (monoklonal) |
| XI | Antikörper | Anti-CDE (monoklonal) |
| XII | Antikörper | Anti-Erythrozyten (polyklonal) |

Indikatorzone VI ist die Kontrolle (ctl) für die Bestimmung von Antikörpern gegen infektiöse Agenzien und enthält anti-human IgG/IgM Antikörper. Indikatorzone XII ist die Kontrolle (ctl) für die Blutgruppenbestimmung und enthält polyklonale anti-Erythrozyten Antikörper. Sie befindet sich distal angeordnet zu allen übrigen Indikatorzonen.

In Fig. 10 wird beispielhaft eine perspektivische Darstellung einer erfindungsgemäßen Vorrichtung für Lateral-Fluss-Tests für die simultane Durchführung von Blutgruppenbestimmung und dem Nachweis von Antikörpern gegen thrombozytäre Antigene, gezeigt. Im vorliegenden Beispiel besteht die Vorrichtung aus einer Trägerschicht 1, der porösen Membran 2, dem Absorptions-Pad 3 und dem zweidimensionalen, in Stegform ausgeführten Dichtelement 4. Dabei ist die poröse Membran 2 auf der mit einem drucksensitiven Acrylatklebstoff versehenen Trägerschicht 1 fixiert. Ebenso ist das Absorptions-Pad 3 auf der Trägerschicht 1 fixiert, wobei ein Teil des Absorptions-Pad 3 mit der porösen Membran 2 überlappt. Das auf der Oberseite der porösen Membran 2 fixierte Dichtelement 4 separiert die Aufgabezone 5 von der übrigen Membranfläche und ermöglicht die gerichtete Verteilung von Probenflüssigkeit und Testreagenzien in die poröse Membran 2. Zwischen der Aufgabezone 5 und dem Bereich der porösen Membran 2, der mit dem Absorptions-Pad 3 in Kontakt steht, ist der Indikatorzonenbereich 6 angeordnet. Dieser wird aus diagonal versetzten, in definierten X- und Y-Positionen angeordneten, punktförmigen Indikatorzonen I-IX gebildet, wobei die Indikatorzonen I-III den Indikatorzonenbereich "Nachweis von Antikörpern gegen thrombozytäre Antigene" und die Indikatorzonen IV-IX den Indikatorzonenbereich "Blutgruppenbestimmung" umfassen und aus den folgenden Bindungselementen bestehen:

| Indikatorzone | Bindungselement | Spezifikation |
|---|---|---|
| | | |
| *Indikatorzonenbereich: Nachweis von Antikörpern gegen thrombozytäre Antigene* | | |
| | | |
| I | Membranproteine | Thrombozyten, HPA 1bb3aa5bb |
| II | Membranproteine | Thrombozyten, HPA 1aa3bb5aa |
| III | Antikörper | Anti-human IgG/IgM |
| | | |
| *Indikatorzonenbereich: Blutgruppenbestimmung* | | |
| | | |
| IV | Antikörper | Anti-A (monoklonal) |
| V | Antikörper | Anti-B (monoklonal) |
| VI | Antikörper | Anti-AB (monoklonal) |
| VII | Antikörper | Anti-D (monoklonal) |
| VIII | Antikörper | Anti-CDE (monoklonal) |
| IX | Antikörper | Anti-Erythrozyten (polyklonal) |

Indikatorzone III ist die Kontrolle (ctl) für den Nachweis von Antikörpern gegen thrombozytäre Antigene und enthält anti-human IgG/IgM Antikörper. Indikatorzone IX ist die Kontrolle (ctl) für die Blutgruppenbestimmung und enthält polyklonale anti-Erythrozyten Antikörper. Sie befindet sich distal angeordnet zu allen übrigen Indikatorzonen.

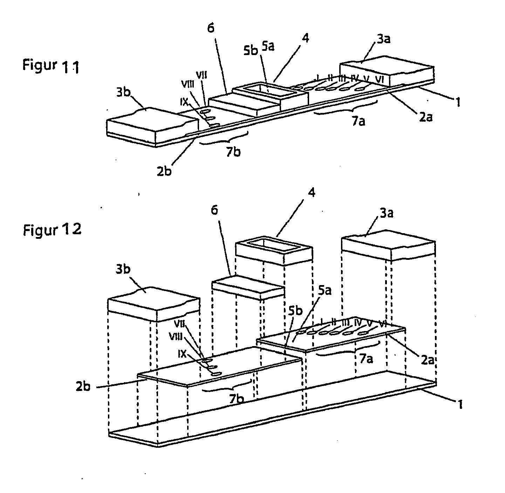

In Fig. 11 wird beispielhaft eine perspektivische Darstellung einer erfindungsgemäßen Vorrichtung für Lateral-Fluss-Tests mit bidirektionalem Fluss für die simultane Bestimmung von Blutgruppenbestimmung und Serumgegenprobe gezeigt. Im vorliegenden Beispiel besteht die Vorrichtung aus einer Trägerschicht 1, einer porösen Membran 2a für die Blutgruppenbestimmung, einer von Membran 2a sich unterscheidenden porösen Membran 2b für die Serumgegenprobe, den Absorptions-Pads 3a und 3b, einem dreidimensionalen, in Trogform ausgeführten Dichtelement 4, und einem Konjugat-Pad 6. Dabei sind die porösen Membranen 2a und 2b auf der mit einem drucksensitiven Acrylatklebstoff versehenen Trägerschicht 1 fixiert. Ebenso sind die Absorptions-Pads 3a und 3b auf der Trägerschicht 1 fixiert, wobei je ein Teil der Absorptions-Pads 3a und 3b mit den porösen Membranen 2a und 2b überlappen. Das auf der Oberseite der porösen Membran 2a und 2b fixierte Dichtelement 4 separiert die Aufgabezonen 5a bzw. 5b von den übrigen Membranflächen und ermöglicht die gerichtete Verteilung von Probenflüssigkeit und Testreagenzien in die porösen Membranen 2a und 2b. Zwischen der Aufgabezone 5a und dem Bereich der porösen Membran 2a, der mit dem Absorptions-Pad 3a in Kontakt steht, ist der Indikatorzonenbereich 7a (Blutgruppenbestimmung) angeordnet. Dieser wird aus diagonal versetzten, in definierten X- und Y-Positionen angeordneten, punktförmigen Indikatorzonen I - VI gebildet, wobei die Indikatorzonen des Indikatorzonenbereiches 7a aus den folgenden Bindungselementen bestehen:

| Indikatorzone | Bindungselement | Spezifikation |
|---|---|---|
| I | Antikörper | Anti-A (monoklonal) |
| II | Antikörper | Anti-B (monoklonal) |
| III | Antikörper | Anti-AB (monoklonal) |
| IV | Antikörper | Anti-D (monoklonal) |
| V | Antikörper | Anti-CDE (monoklonal) |
| VI | Antikörper | Anti-Erythrozyten (polyklonal) |

Die Indikatorzone VI ist die Kontrolle (ctl) für die Blutgruppenbestimmung und enthält polyklonale anti-Erythrozyten Antikörper. Sie befindet sich distal angeordnet zu den Indikatorzonen I-V.

Zwischen der Aufgabezone 5b und dem Bereich der porösen Membran 2b, der mit dem Absorptions-Pad 3b in Kontakt steht, ist der Indikatorzonenbereich 7b (Serumgegenprobe) angeordnet. Dieser wird aus diagonal versetzten, in definierten X- und Y-Positionen angeordneten, punktförmigen Indikatorzonen VII - IX gebildet, wobei die Indikatorzonen des Indikatorzonenbereiches 7b aus den folgenden Bindungselementen bestehen:

| Indikatorzone | Bindungselement | Spezifikation |
|---|---|---|
| VII | Erythrozyten-Ghosts | Blutgruppe A |
| VIII | Erythrozyten-Ghosts | Blutgruppe B |
| IX | Erythrozyten-Ghosts | Blutgruppe 0 (Kontrolle) |

In Fig. 12 wird eine Explosionsdarstellung der in Fig. 11 dargestellten erfindungsgemäßen Vorrichtung für Lateral-Fluss-Tests mit bidirektionalem Fluss gezeigt, die aus den Komponenten Trägerschicht 1, einer porösen Membran 2a für die Blutgruppenbestimmung, einer von Membran 2a sich unterscheidenden porösen Membran 2b für die Serumgegenprobe, den Absorptions-Pads 3a und 3b, einem dreidimensionalen, in Trogform ausgeführten Dichtelement 4, und einem Konjugat-Pad 6 bestehen. Die Probenaufgabezone erstreckt sich über beide porösen Membranen mit der Probenaufgabezone 5a der Membran 2a und der Probenaufgabezone 5b der Membran 2b und wird durch das in Trogform ausgeführte Dichtelement 4 von den übrigen Flächen der Membranen 2a und 2b separiert. Die Membran 2a enthält den Indikatorzonenbereich 7a mit den von proximal nach distal diagonal versetzt angeordneten Indikatorzonen I-VI, während die Membran 2b den Indikatorzonenbereich 7b mit den von proximal nach distal diagonal versetzt angeordneten Indikatorzonen VII-IX enthält.

### Beispiele

### Beispiel 1: Simultane Blutgruppenbestimmung (direkter Assay) und Serumgegenprobe (direkter Bindungsassay)

### Herstellung der Teststreifen:

Die Teststreifen bestehen aus einer Aufgabezone, einem Indikatorzonenbereich und einem Absorptionsbereich. Membranen der Sorte Millipore HiFlow Plus 065 werden in Streifen auf eine Größe von 15 mm x 35 mm (Breite/Länge; x/y) zurechtgeschnitten und auf eine Trägerschicht (Backing Sheet, z. B. von G&L) aufgeklebt. Diagonal versetzt werden im Indikatorzonenbereich, der sich in die Indikatorzonenbereiche "Serumgegenprobe" (proximal zur Aufgabezone angeordnet) und Blutgruppenbestimmung (distal zur Aufgabezone angeordnet) aufteilt, je 0,2 µl Punkte der verschiedenen Bindungselemente unter Verwendung eines Dispensers, z.B. AD3200 (Biodot) aufgetragen:

Indikatorzonenbereich "Serumgegenprobe" - Suspensionen von Erythrozyten-Ghosts der Spezifikation Blutgruppe A1, Blutgruppe A2, Blutgruppe B und Blutgruppe 0 (hergestellt aus Erythrozytenkonzentraten) sowie Anti-human IgG/IgM Antikörper (Goat anti human IgG, Goat anti human IgM, Sigma, I-3382, I-0759) als Kontrolle; Indikatorzonenbereich "Blutgruppenbestimmung" - Anti-A Antikörper - Klon Birma-1 (Serologicals, TLJ0105); Anti-B Antikörper - Klon ES-4 (Serologicals, NCA0201); Anti-AB Antikörper - Klone AB6, AB26, AB92 (Medion Diagnostics, 010062); anti-Erythrozyten Antikörper (Rabbit IgG Fraction of anti Human RBC, Rockland, 209-4139).

Die Positionierung der Bindungselemente des Indikatorzonenbereiches "Serumgegenprobe" startet mit Erythrozyten-Ghosts der Spezifikation Blutgruppe A1 in Position x=2,5 mm/ y=10 mm. Alle anderen Bindungselemente werden iterierend in Abständen von x=2,5 mm/ y=1,5 mm zur Position der Erythrozyten-Ghosts der Spezifikation Blutgruppe A1 dispensiert. Die Erythrozyten-Ghosts werden als 0.1-0.5 %ige (v/v) Suspensionen in 15 mM Kaliumphosphatpuffer pH 7,5, 10% (v/v) Methanol, die anti-human IgG/IgM Antikörper als 1:1 Gemisch in Konzentration von 50 µg/ml in 15 mM Kaliumphosphatpuffer pH 7,5, 10% (v/v) Methanol dispensiert.

Die Positionierung der Bindungselemente des Indikatorzonenbereiches "Blutgruppenbestimmung" startet mit dem Anti-A Antikörper in Position x=3 mm/y=20 mm. Alle anderen Bindungselemente werden iterierend in Abständen von x=3 mm/ y=1,5 mm zur Position des anti-A Antikörpers dispensiert. Die Verdünnungen der Antikörper erfolgen in 15 mM Kaliumphosphatpuffer pH 7,5, 10% (v/v) Methanol) wie folgt: Anti-A Antikörper 1:3, anti-B Antikörper 1:2, anti-AB Antikörper 1:4, anti-RBC Antikörper 1:3.

Die Membranen werden nach dem Dispensieren der Bindungselemente für 20 min bei 40°C getrocknet und anschließend bei konstanter Luftfeuchte bis zur Testdurchführung aufbewahrt. Am zur Aufgabezone distalen Ende wird ein mit der Membran um 3 mm überlappendes 15x15 mm großes Absorptions-Pad (Schleicher & Schüll, 300) aufgeklebt. Die Aufgabezone wird durch Aufkleben eines 1-2 mm breiten Klebestreifens (Tesa 4124) in Position y=5 mm über die gesamte Membranbreite von der übrigen Membran separiert.

### Testansatz:

Als Blutproben werden antikoagulierte Vollblute verwendet. Für den eigentlichen Test werden 100 µl unverdünntes bzw. 1:3 oder 1:6 in Verdünnungspuffer (EnlisstII, Medion Diagnostics oder Diluent 1, DiaMed) verdünntes Blut in die Aufgabezone aufgetragen. Wenn das Blut die Aufgabezone verlassen hat, wird zweimalig mit 100 µl EnlisstII gewaschen, um ungebundene Erythrozyten aus der Membran zu entfernen. Im Anschluß werden 50 µl anti-IgG/A/M konjugierte Goldpartikel (20 bis 40 nm, Arista Biologicals, CGIGA-0800, CGIGG-0800, CGIGM-0800), 1:10 (v/v) verdünnt in TBS , 0.08 % Gelatine, 0.5 % Albumin, auf die Aufgabezone aufgetragen. Anstelle der Goldpartikel können auch farbige, 100 bis 400 nm Polystyrolpartikel verwendet werden, z.B. von Merck Eurolab France/Estapor. Wenn die Goldpartikel die Aufgabezone verlassen haben, wird die Membran nochmals ein- oder zweimalig mit 100 µl EnlisstII gewaschen.

### Ergebnis:

(a) Kontrollen: Der Test ist gültig, wenn die anti-RBC Kontrolle (Indikatorzone IX, Indikatorzonenbereich "Blutgruppenbestimmung") ein deutlich positives Signal (Roter Punkt) zeigt und wenn die anti-IgG/IgM-Kontrolle (Indikatorzone V, Indikatorzonenbereich "Serumgegenprobe") charakteristisch purpurn (Goldpartikel) oder in der Farbe der verwendeten Polystyrolpartikel gefärbt ist.
(b) Testergebnisse: Je nach Anwesenheit oder Abwesenheit der jeweiligen Blutgruppenantigene erscheinen an den entsprechenden Positionen im Indikatorzonenbereich "Blutgruppenbestimmung" rote Punkte (positiv) oder die fast weisse Hintergrundfärbung der Membran (negativ). Die korrespondierenden Isoagglutinine sind im Indikatorzonenbereich "Serumgegenprobe" durch das charakteristische Purpur der Goldpartikel als purpurfarbene Punkte oder in der Farbe der verwendeten Polystyrolpartikel erkennbar. Bei Abwesenheit eines Isoagglutinins sind keine sich vom Hintergrund unterscheidenden Signale in diesen Positionen zu erkennen.

### Beispiel 2: Simultanbestimmung von Blutgruppen (direkter Assay) und Serumgegenprobe (Kompetitionsassay)

### Herstellung der Teststreifen:

Die Teststreifen bestehen aus einer Aufgabezone, zwei Indikatorzonenbereichen zu beiden Seiten der Aufgabezone und zwei Absorptionsbereichen. Membranen der Sorte Millipore HiFlow Plus 065 und HiFlow Plus 140 werden in Streifen auf eine Größe von 15 mm x 20 mm (Breite/Länge; x/y) zurechtgeschnitten und nebeneinander auf eine Trägerschicht (Backing Sheet, z. B. von G&L) aufgeklebt. Auf der HiFlow Plus 140 Membran wird zusätzlich ein Konjugat Pad, in das anti-A/anti-B/anti-H konjugierte Goldpartikel eingetrocknet sind, so aufgebracht, dass es sich zwischen Dichtelement (Klebestreifen) und Indikatorzonen befindet. Anstelle der Goldpartikel können auch farbige, 100 bis 400 nm Polystyrolpartikel verwendet werden, z.B. von Merck Eurolab France/Estapor. Diagonal versetzt werden im Indikatorzonenbereich "Blutgruppenbestimmung", der sich auf der HiFlow Plus 065 Membran befindet, je 0,2 µl Punkte folgender Bindungselemente unter Verwendung eines Dispensers, z.B. AD3200 (Biodot) aufgetragen: Anti-A Antikörper - Klon Birma-1 (Serologicals, TLJ0105); Anti-B Antikörper - Klon ES-4 (Serologicals, NCA0201); Anti-AB Antikörper - Klone AB6, AB26, AB92 (Medion Diagnostics, 010062); anti-Erythrozyten Antikörper (Rabbit IgG Fraction of anti Human RBC, Rockland, 209-4139).

In gleicher Weise werden im Indikatorzonenbereich "Serumgegenprobe", der sich auf der HiFlow Plus 140 Membran befindet, - Suspensionen von Erythrozyten-Ghosts der Blutgruppe A, Blutgruppe B und Blutgruppe 0 (hergestellt aus Erythrozytenkonzentraten) aufgetragen.

Die Positionierung der Bindungselemente des Indikatorzonenbereiches "Serumgegenprobe" startet mit Erythrozyten-Ghosts der Spezifikation Blutgruppe A in Position x=3 mm/ y=10 mm. Alle anderen Bindungselemente werden iterierend in Abständen von x=3 mm/ y=1,5 mm zur Position der Erythrozyten-Ghosts der Spezifikation Blutgruppe A dispensiert. Die Erythrozyten-Ghosts werden als 0.1-0.5 %ige (v/v) Suspensionen in 15 mM Kaliumphosphatpuffer pH 7,5, 10% (v/v) Methanol dispensiert.

Die Positionierung der Bindungselemente des Indikatorzonenbereiches "Blutgruppenbestimmung" startet mit dem Anti-A Antikörper in Position x=2,5 mm/y=20 mm. Alle anderen Bindungselemente werden iterierend in Abständen von x=2 mm/ y=1,5 mm zur Position des anti-A Antikörpers dispensiert. Die Verdünnungen der Antikörper erfolgen in 15 mM Kaliumphosphatpuffer pH 7,5, 10% (v/v) Methanol) wie folgt: Anti-A Antikörper 1:3, anti-B Antikörper 1:2, anti-AB Antikörper 1:4, anti-RBC Antikörper 1:3.

Die Membranen werden nach dem Dispensieren der Bindungselemente für 20 min bei 40°C getrocknet und anschließend bei konstanter Luftfeuchte bis zur Testdurchführung aufbewahrt. An den beiden zur Aufgabezone distalen Enden wird ein mit der Membran um 3 mm überlappendes 15x15 mm großes Absorptions-Pad (Schleicher & Schüll, 300) aufgeklebt. Die Aufgabezone wird durch Aufkleben eines 1-2 mm breiten Klebestreifens (Tesa 4124) in Position y=5 mm über die gesamte Membranbreite von der übrigen Membran separiert.

### Testansatz:

Als Blutproben werden antikoagulierte Vollblute verwendet. Für den eigentlichen Test werden 100 µl unverdünntes bzw. 1:3 oder 1:6 in Verdünnungspuffer (EnlisstII, Medion Diagnostics oder Diluent 1, DiaMed) verdünntes Blut in die Aufgabezone aufgetragen. Wenn das Blut die Aufgabezone verlassen hat, wird zweimalig mit 100 µl EnlisstII gewaschen, um ungebundene Erythrozyten aus der Membran zu entfernen.

### Ergebnis:

(a) Kontrollen: Der Test ist gültig, wenn die anti-RBC Kontrolle (Indikatorzonenbereich "Blutgruppenbestimmung") ein deutlich positives Signal (Roter Punkt) zeigt und wenn die Erythrozyten-Blutgruppe 0 Kontrolle (Indikatorzonenbereich "Serumgegenprobe") charakteristisch purpurn (Goldpartikel) oder in der Farbe der verwendeten Polystyrolpartikel gefärbt ist.
(b) Testergebnisse: Je nach Anwesenheit oder Abwesenheit der jeweiligen Blutgruppenantigene erscheinen an den entsprechenden Positionen im Indikatorzonenbereich "Blutgruppenbestimmung" rote Punkte (positiv) oder die fast weisse Hintergrundfärbung der Membran (negativ). Die korrespondierenden Isoagglutinine sind im Indikatorzonenbereich "Serumgegenprobe" durch das Fehlen der entsprechenden Bande gekennzeichnet. Bei Abwesenheit eines Isoagglutinins ist die entsprechende Bande charakteristisch angefärbt.

### Beispiel 3: Simultane Blutgruppenbestimmung, Serumgegenprobe und Antikörpersuch-Test bei Empfängern

### Herstellung der Teststreifen:

Die Teststreifen bestehen aus einer Aufgabezone, einem Indikatorzonenbereich und einem Absorptionsbereich. Membranen der Sorte Millipore HiFlow Plus 065 werden in Streifen auf eine Größe von 20 mm x 35 mm (Breite/Länge; x/y) zurechtgeschnitten und auf eine Trägerschicht (Backing Sheet, z. B. von G&L) aufgeklebt. Diagonal versetzt werden im Indikatorzonenbereich, der sich in die Indikatorzonenbereiche "Serumgegenprobe/ Antikörpersuche" (proximal zur Aufgabezone angeordnet) und "Blutgruppenbestimmung" (distal zur Aufgabezone angeordnet) aufteilt, je 0,2 µl Punkte der verschiedenen Bindungselemente unter Verwendung eines Dispensers, z.B. AD3200 (Biodot) aufgetragen:

Indikatorzonenbereich "Serumgegenprobe/Antikörpersuche" - Suspensionen von Erythrozyten-Ghosts der Spezifikation Blutgruppe A1, Blutgruppe A2, Blutgruppe B, Blutgruppe 0, Blutgruppe 0 Rh-Formel R₁R₁^{W} (Suchzelle 1), Blutgruppe 0 Rh-Formel R₂R₂ (Suchzelle 2), Blutgruppe 0 Rh-Formel R₁R₁ (Suchzelle 3), sowie Anti-human IgG/IgM (Goat anti human IgG, Goat anti human IgM, Sigma, I-3382, 1-0759) als Kontrolle; Indikatorzonenbereich "Blutgruppenbestimmung" - Anti-A Antikörper - Klon Birma-1 (Serologicals, TLJ0105); Anti-B Antikörper - Klon ES-4 (Serologicals, NCA0201); Anti-AB Antikörper- (Klone AB6, AB26, AB92 (Medion Diagnostics, 010062); anti-Erythrozyten Antikörper (Rabbit IgG Fraction of anti Human RBC, Rockland, 209-4139).

Die Positionierung der Bindungselemente des Indikatorzonenbereiches "Serumgegenprobe" startet mit Erythrozyten-Ghosts der Spezifikation Blutgruppe A1 in Position x=3 mm/ y=10 mm, die Positionierung der Erythrozyten-Ghosts der Spezifikation Blutgruppe A2, B, 0 folgt iterierend in Abständen von x=2 mm/ y=1,5 mm. Die Positionierung der Erythrozyten-Ghosts der Suchzelle 1 erfolgt in Position x=11 mm/ y=10 mm, die Positionierung der Erythrozyten-Ghosts der Suchzellen 2 und 3 sowie des humanen IgG/IgM iterierend in Abständen von x=2 mm/y=1,5 mm. Die Erythrozyten-Ghosts werden als 0.1-0.5%ige (v/v) Suspensionen in 15 mM Kaliumphosphatpuffer pH 7,5, 10% (v/v) Methanol, die anti-human IgG/IgM Antikörper als 1:1 Gemisch in Konzentration von 50 µg/ml in 15 mM Kaliumphosphatpuffer pH 7,5, 10% (v/v) Methanol dispensiert.

Die Positionierung der Bindungselemente des Indikatorzonenbereiches "Blutgruppenbestimmung" startet mit dem Anti-A Antikörper in Position x=4. mm/y=20 mm. Alle anderen Bindungselemente werden iterierend in Abständen von x=4 mm/ y=1,5 mm zur Position des anti-A Antikörpers dispensiert. Die Verdünnungen der Antikörper erfolgen in 15 mM Kaliumphosphatpuffer pH 7,5, 10% (v/v) Methanol) wie folgt: Anti-A Antikörper 1:3, anti-B Antikörper 1:2, anti-AB Antikörper 1:4, anti-RBC Antikörper 1:3.

Die Membranen werden nach dem Dispensieren der Bindungselemente für 20 min bei 40°C getrocknet und anschließend bei konstanter Luftfeuchte bis zur Testdurchführung aufbewahrt. Am zur Aufgabezone distalen Ende wird ein mit der Membran um 3 mm überlappendes 20x15 mm großes Absorptions-Pad (Schleicher & Schüll, 300) aufgeklebt. Die Aufgabezone wird durch Aufkleben eines 1-2 mm breiten Klebestreifens (Tesa 4124) in Position y=5 mm über die gesamte Membranbreite von der übrigen Membran separiert.

### Testansatz:

Als Blutproben werden antikoagulierte Vollblute verwendet. Für den eigentlichen Test werden 120 µl unverdünntes bzw. 1:3 oder 1:6 in Verdünnungspuffer (EnlisstII, Medion Diagnostics oder Diluent 1, DiaMed) verdünntes Blut in die Aufgabezone aufgetragen. Wenn das Blut die Aufgabezone verlassen hat, wird zweimalig mit 120 µl EnlisstII gewaschen, um ungebundene Erythrozyten aus der Membran zu entfernen. Im Anschluß werden 75 µl anti-IgG/A/M konjugierte Goldpartikel (20 bis 40 nm, Arista Biologicals, CGIGA-0800, CGIGG-0800, CGIGM-0800), 1:10 (v/v) verdünnt in TBS, 0.08 % Gelatine, 0.5 % Albumin, auf die Aufgabezone aufgetragen. Anstelle der Goldpartikel können auch farbige, 100 bis 400 nm Polystyrolpartikel verwendet werden, z.B. von Merck Eurolab France/Estapor. Wenn die Goldpartikel die Aufgabezone verlassen haben, wird die Membran nochmals ein- oder zweimalig mit 120 µl EnlisstII gewaschen.

### Ergebnis:

(a) Kontrollen: Der Test ist gültig, wenn die anti-RBC Kontrolle (Indikatorzone XII, Indikatorzonenbereich "Blutgruppenbestimmung'') ein deutlich positives Signal (Roter Punkt) zeigt und wenn die anti-IgG/IgM-Kontrolle (Indikatorzone VIII, Indikatorzonenbereich "Serumgegenprobe/Antikörpersuche'') charakteristisch pupurn (Goldpartikel) oder in der Farbe der verwendeten Polystyrolpartikel gefärbt ist. Die anti-IgG/IgM-Kontrolle muss in jedem Fall angefärbt sein, so dass bei einer Person mit Blutgruppe AB, die keine irregulären Antikörper hat, die Anfärbung dieser Indikatorzone bei völliger Abwesenheit anderer Signale im Indikatorzonenbreich "Serumgegenprobe/Antikörpersuche" auf eine korrekte Testdurchführung verweist. Die Indikatorzone IV (Erythrozyten-Ghosts Blutgruppe 0) ist eine negative Kontrolle für Isoagglutinine. Eine Anfärbung dieser Indikatorzone bedeutet, dass neben Isoagglutininen auch irreguläre Antikörper vorhanden sein müssen, d. h. dass mindestens eine der drei Indikatorzonen V, VI, VII ebenfalls angefärbt sein muss. Ist dies nicht der Fall, so ist der Test ungültig.
(b) Testergebnisse: Je nach Anwesenheit oder Abwesenheit der jeweiligen Blutgruppenantigene erscheinen an den entsprechenden Positionen im Indikatorzonenbereich "Blutgruppenbestimmung" rote Punkte (positiv) oder die fast weisse Hintergrundfärbung der Membran (negativ). Die korrespondierenden Isoagglutinine sind im Indikatorzonenbereich "Serumgegenprobe" durch das charakteristische Purpur der Goldpartikel als purpurfarbene Punkte oder in der Farbe der verwendeten Polystyrolpartikel erkennbar. Bei Abwesenheit eines Isoagglutinins sind keine sich vom Hintergrund unterscheidenden Signale in diesen Positionen zu erkennen. Bei Vorhandensein eines irregulären Antikörpers sind ein, zwei oder alle drei der Indikatorzonen mit den Erythrozyten-Ghosts der Suchzellen 1, 2 oder 3 durch das charakteristische Purpur der Goldpartikel angefärbt oder in der Farbe der verwendeten Polystyrolpartikel erkennbar.

### Beispiel 4: Simultane Blutgruppenbestimmung, Serumgegenprobe und Antikörpersuch-Test für Spender

### Herstellung der Teststreifen:

Der grundsätzliche Aufbau der Teststreifen entspricht dem Teststreifenaufbau in Beispiel 2. Das Format der Millipore HiFlow Plus 065 Membran beträgt 15 mm x 35 mm (Breite/Länge; x/y). Der Indikatorzonen des Indikatorzonenbereiches "Blutgruppenbestimmung" entsprechen dem Beispiel 2. Im Indikatorzonenbereich "Serumgegenprobe/Antikörpersuche" werden je 0,2 µl Punkte der folgenden Bindungselemente unter Verwendung eines Dispensers, z.B. AD3200 (Biodot) dispensiert:

Suspensionen von Erythrozyten-Ghosts der Spezifikation Blutgruppe A1, Blutgruppe A2, Blutgruppe B, Blutgruppe 0, Erythrozytenghosts aus einem Gemisch der Suchzellen 1-3 (siehe Beispiel 2), sowie Anti-human IgG/IgM (Goat anti human IgG, Goat anti human IgM, Sigma, I-3382, I-0759) als Kontrolle.

Die Positionierung der Bindungselemente des Indikatorzonenbereiches "Serumgegenprobe/Antikörpersuche" startet mit Erythrozyten-Ghosts der Spezifikation Blutgruppe A1 in Position x=2,5 mm/ y=10 mm, die Positionierung der Erythrozyten-Ghosts der Spezifikation Blutgruppe A2, B, 0 iterierend in Abständen von x=2 mm/ y=1,5 mm. Die Positionierung der Erythrozyten-Ghosts des Gemisches der Suchzellen 1-3 erfolgt in Position x=10,5 mm/ y=13 mm, die des humanen IgG/IgM in Position x=12,5 mm/ y=14,5 mm. Die Erythrozyten-Ghosts werden als 0.1-0.5%ige (v/v) Suspensionen in 1 mM Kaliumphosphatpuffer pH 7,5, 10% (v/v) Methanol, die anti-human IgG/IgM Antikörper als 1:1 Gemisch in Konzentration von 50 µg/ml in 15 mM Kaliumphosphatpuffer pH 7,5, 10% (v/v) Methanol dispensiert.

Die Positionierung der Bindungselemente des Indikatorzonenbereiches "Blutgruppenbestimmung" startet mit dem Anti-A Antikörper in Position x=3 mm/y=20 mm. Alle anderen Bindungselemente des Indikatorzonenbereiches werden iterierend in Abständen von x=3 mm/ y=1,5 mm zur Position des anti-A Antikörpers dispensiert. Die Verdünnungen der Antikörper erfolgen in 15 mM Kaliumphosphatpuffer pH 7,5, 10% (v/v) Methanol) wie folgt: Anti-A Antikörper 1:3, anti-B Antikörper 1:2, anti-AB Antikörper 1:4, anti-RBC Antikörper 1:3.

Die Membranen werden nach dem Dispensieren der Bindungselemente für 20 min bei 40°C getrocknet und anschließend bei konstanter Luftfeuchte bis zur Testdurchführung aufbewahrt. Am zur Aufgabezone distalen Ende wird ein mit der Membran um 3 mm überlappendes 15x15 mm großes Absorptions-Pad (Schleicher & Schüll, 300) aufgeklebt. Die Aufgabezone wird durch Aufkleben eines 1-2 mm breiten Klebestreifens (Tesa 4124) in Position y=6 mm über die gesamte Membranbreite von der übrigen Membran separiert.

### Testansatz:

Der Testansatz entspricht dem Ansatz in Beispiel 1.

### Ergebnis:

(a) Kontrollen: Der Test ist gültig, wenn die anti-RBC Kontrolle (Indikatorzone X, Indikatorzonenbereich "Blutgruppenbestimmung") ein deutlich positives Signal (Roter Punkt) zeigt und wenn die anti-IgG/IgM-Kontrolle (Indikatorzone VI, Indikatorzonenbereich "Serumgegenprobe/ Antikörpersuche") charakteristisch pupurn (Goldpartikel) oder in der Farbe der verwendeten Polystyrolpartikel gefärbt ist. Die anti-IgG/IgM-Kontrolle muss in jedem Fall angefärbt sein, so dass bei einer Person mit Blutgruppe AB, die keine irregulären Antikörper hat, die Anfärbung dieser Indikatorzone bei völliger Abwesenheit anderer Signale im Indikatorzonenbreich "Serumgegenprobe/Antikörpersuche" auf eine korrekte Testdurchführung verweist. Die Indikatorzone IV (Erythrozyten-Ghosts Blutgruppe 0) ist eine negative Kontrolle. Eine Anfärbung dieser Indikatorzone bedeutet, dass neben Isoagglutininen auch irreguläre Antikörper vorhanden sein müssen, d. h. dass mindestens eine der drei Indikatorzonen V, VI, VII ebenfalls angefärbt sein muss. Ist dies nicht der Fall, so ist der Test ungültig.
(b) Testergebnisse: Je nach Anwesenheit oder Abwesenheit der jeweiligen Blutgruppenantigene erscheinen an den entsprechenden Positionen im Indikatorzonenbereich "Blutgruppenbestimmung" rote Punkte (positiv) oder die fast weisse Hintergrundfärbung der Membran (negativ). Die korrespondierenden Isoagglutinine sind im Indikatorzonenbereich "Serumgegenprobe" durch das charakteristische Purpur der Goldpartikel als purpurfarbene Punkte oder in der Farbe der verwendeten Polystyrolpartikel erkennbar. Bei Abwesenheit eines Isoagglutinins sind keine sich vom Hintergrund unterscheidenden Signale in diesen Positionen zu erkennen. Bei Vorhandensein eines irregulären Antikörpers sind ein, zwei oder alle drei der Indikatorzonen mit den Erythrozyten-Ghosts der Suchzellen 1, 2 oder 3 durch das charakteristische Purpur der Goldpartikel angefärbt oder in der Farbe der verwendeten Polystyrolpartikel erkennbar.

### Beispiel 5: Simultane Blutgruppenbestimmung und Nachweis von Infektionsmarkern

### Herstellung der Teststreifen:

Die Teststreifen bestehen aus einer Aufgabezone, einem Indikatorzonenbereich und einem Absorptionsbereich. Membranen der Sorte Millipore HiFlow Plus 065 werden in Streifen auf eine Größe von 15 mm x 35 mm (Breite/Länge; x/y) zurechtgeschnitten und auf eine Trägerschicht (Backing Sheet z. B. von G&L) aufgeklebt. Diagonal versetzt werden im Indikatorzonenbereich, der sich in die Indikatorzonenbereiche "Nachweis von Infektionsmarkern" (proximal zur Aufgabezone angeordnet) und "Blutgruppenbestimmung" (distal zur Aufgabezone angeordnet) aufteilt, je 0,2 *µ*l Punkte der verschiedenen Bindungselemente unter Verwendung eines Dispensers, z.B. AD3200 (Biodot) aufgetragen:

Indikatorzonenbereich "Nachweis von Infektionsmarkern" - Lösungen rekombinanter Antigene (Syphilis; TpN 15, TpN 17, TpN 47), synthetischer Peptide aus Sequenzen der Glykoproteine gp-14, gp-41 (HIV-1; HIV-O) und gp-36 (HIV-2), rekombinante HCV Antigene (C-100, C-200, C33c, C22), monoklonale Antikörper (HBsAg) sowie Anti-human IgG/IgM (Goat anti human IgG, Goat anti human IgM, Sigma, I-3382, I-0759) als Kontrolle; Indikatorzonenbereich "Blutgruppenbestimmung" - Anti-A Antikörper - Klon Birma-1 (Serologicals, TLJ0105); Anti-B Antikörper - Klon ES-4 (Serologicals, NCA0201); Anti-AB Antikörper - Klone AB6, AB26, AB92 (Medion Diagnostics, 010062); Anti-D Antikörper - Klone LDM3/ESD1 (SNBTS), Anti-CDE Antikörper - Klone MS-24/MS-201/MS 80/MS-258 (Serologicals), Anti-Erythrozyten Antikörper (Rabbit IgG Fraction of anti Human RBC, Rockland, 209-4139).

Die Positionierung der Bindungselemente des Indikatorzonenbereiches "Nachweis von Infektionsmarkern" startet mit synthetischen Peptiden der Spezifität HIV-1 (gp-14, gp-41) in Position x=2,5 mm/ y=10 mm. Alle anderen Bindungselemente werden iterierend in Abständen von x=2 mm/ y=1,5 mm zur Position der Indikatorzone I dispensiert. Die Bindungselemente der Indikatorzonen I-V werden in geeigneten Konzentrationen in 15 mM Kaliumphosphatpuffer pH 7,5, 10% (v/v) Methanol, die anti-human IgG/IgM Antikörper in einer Konzentration von 50 µg/ml in 15 mM Kaliumphosphatpuffer pH 7,5, 10% (v/v) Methanol dispensiert.

Die Positionierung der Bindungselemente des Indikatorzonenbereiches "Blutgruppenbestimmung" startet mit dem Anti-A Antikörper in Position x=2,5 mm/y=20 mm. Alle anderen Bindungselemente des Indikatorzonenbereiches werden iterierend in Abständen von x=2 mm/ y=1,5 mm zur Position des anti-A Antikörpers dispensiert. Die Verdünnungen der Antikörper erfolgen in 15 mM Kaliumphosphatpuffer pH 7,5, 10% (v/v) Methanol) wie folgt: Anti-A Antikörper 1:3, anti-B Antikörper 1:2, anti-AB Antikörper 1:4, anti-RBC Antikörper 1:3.

Die Membranen werden nach dem Dispensieren der Bindungselemente für 20 min bei 40°C getrocknet und anschließend bei konstanter Luftfeuchte bis zur Testdurchführung aufbewahrt. Am zur Aufgabezone distalen Ende wird ein mit der Membran um 3 mm überlappendes 15x15 mm großes Absorptions-Pad (Schleicher & Schüll, 300) aufgeklebt. Die Aufgabezone wird durch Aufkleben eines 1-2 mm breiten Klebestreifens (Tesa 4124) in Position y=6 mm über die gesamte Membranbreite von der übrigen Membran separiert.

### Testansatz:

Als Blutproben werden antikoagulierte Vollblute verwendet. Für den eigentlichen Test werden 100 µl unverdünntes bzw. 1:3 bzw. 1:6 in Verdünnungspuffer (EnlisstII, Medion Diagnostics oder Diluent 1, DiaMed) verdünntes Blut in die Aufgabezone aufgetragen. Wenn das Blut die Aufgabezone verlassen hat, wird zweimalig mit 100 µl EnlisstII gewaschen, um ungebundene Erythrozyten aus der Membran zu waschen.

Im Anschluß werden 50 µl eines Gemisches aus anti-IgG/A/M konjugierten Goldpartikeln (20 bis 40 nm, Arista Biologicals, CGIGA-0800, CGIGG-0800, CGIGM-0800), 1:10 (v/v) verdünnt in TBS , 0.08 % Gelatine, 0.5 % Albumin, sowie anti-HbsAg konjugierten Goldpartikeln (Arista Biologicals, ABHBS-0500) in geeigneter Verdünnung auf die Aufgabezone aufgetragen. Anstelle der Goldpartikel können auch farbige, 100 bis 400 nm Polystyrolpartikel verwendet werden, z.B. von Merck Eurolab France/Estapor. Wenn diese die Aufgabezone verlassen haben, wird die Membran nochmals ein- oder zweimalig mit 100 µl EnlisstII gewaschen.

### Ergebnis:

Der Test ist gültig, wenn die anti-RBC Kontrolle (Indikatorzone XII, Indikatorzonenbereich "Blutgruppenbestimmung") ein deutlich positives Signal (Roter Punkt) zeigt und wenn die anti-IgG/IgM-Kontrolle (Indikatorzone VI, Indikatorzonenbereich "Nachweis von Infektionsmarkern") charakteristisch pupurn (Goldpartikel) oder in der Farbe der verwendeten Polystyrolpartikel gefärbt ist. Je nach Anwesenheit oder Abwesenheit der jeweiligen Blutgruppenantigene erscheinen an den entsprechenden Positionen rote Punkte (positiv) oder die fast weisse Hintergrundfärbung der Membran (negativ). Bei Vorhandensein von Antikörpern gegen HIV-1, HIV-2, Syphilis bzw. von Hepatitis B surface Antigen (HBsAg) ist die jeweilige Position durch das für Gold-Partikel charakteristische Purpur angefärbt und als purpurfarbene Punkte erkennbar. Wenn Polystyrolpartikel als Indikator-Partikel verwendet werden, ist die jeweilige Position in der Farbe der verwendeten Polystyrolpartikel gefärbt. Im weitaus häufigsten Fall, nämlich einer negativen Reaktion für alle Infektionsmarker, wird nur die anti-IgG/IgM-Kontrolle (Indikatorzone VI, Indikatorzonenbereich "Nachweis von Infektionsmarkern") angefärbt.

### Beispiel 6: Simultane Blutgruppenbestimmung und Nachweis von Antikörpern gegen thrombozytäre Antigene

### Herstellung der Teststreifen:

Der grundsätzliche Aufbau und das Format der Teststreifen entspricht dem Teststreifenaufbau in Beispiel 4. Der Indikatorzonenbereich untergliedert sich in die Indikatorzonenbereiche "Blutgruppenbestimmung" und "Nachweis von Antikörpern gegen thrombozytäre Antigene". Es werden je 0,2 µl Punkte der folgenden Bindungselemente unter Verwendung eines Dispensers, z.B. AD3200 (Biodot) dispensiert:

Indikatorzonenbereich "Nachweis von Antikörpern gegen thrombozytäre Antigene" - Membranproteine von Thrombozyten der Blutgruppe 0 mit distinkten HPA-Antigenprofilen wie HPA 1bb3aa5bb und HPA 1aa3bb5aa sowie Anti-human IgG/IgM (Goat anti human IgG, Goat anti human IgM, Sigma, I-3382, I-0759) als Kontrolle; Indikatorzonenbereich "Blutgruppenbestimmung" - Anti-A Antikörper - Klon Birma-1 (Serologicals, TLJ0105); Anti-B Antikörper - Klon ES-4 (Serologicals, NCA0201); Anti-AB Antikörper - Klone AB6, AB26, AB92 (Medion Diagnostics, 010062); Anti-D Antikörper - Klone LDM3/ESD1 (SNBTS), Anti-CDE Antikörper - Klone MS-24/MS-201/MS 80/MS-258 (Serologicals), Anti-Erythrozyten Antikörper (Rabbit IgG Fraction of anti Human RBC, Rockland, 209-4139). Als Alternative zu den Membranproteinen der Thrombozyten können auch rekombinante Antigene mit den entsprechenden Merkmalsausprägungen (HPA-Antigenprofile) aufgetragen werden.

Die Positionierung der Bindungselemente des Indikatorzonenbereiches "Nachweis von Antikörpern gegen thrombozytäre Antigene" startet mit Membranproteinen Antigenprofil HPA 1bb3aa5bb in Position x=4 mm/ y=10 mm. Alle anderen Bindungselemente werden iterierend in Abständen von x=3,5 mm/ y=2 mm zur Position der Indikatorzone I dispensiert. Die Bindungselemente der Indikatorzonen I und II werden in geeigneten Konzentrationen in 15 mM Kaliumphosphatpuffer pH 7,5, 10% (v/v) Methanol, die anti-human IgG/IgM Antikörper in einer Konzentration von 50 µg/ml in 15 mM Kaliumphosphatpuffer pH 7,5, 10% (v/v) Methanol dispensiert.

Die Positionierung der Bindungselemente des Indikatorzonenbereiches "Blutgruppenbestimmung" entspricht dem Beispiel 4.

Die Membranen werden nach dem Dispensieren der Bindungselemente für 20 min bei 40°C getrocknet und anschließend bei konstanter Luftfeuchte bis zur Testdurchführung aufbewahrt. Am zur Aufgabezone distalen Ende wird ein mit der Membran um 3 mm überlappendes 15x15 mm großes Absorptions-Pad (Schleicher & Schüll, 300) aufgeklebt. Die Aufgabezone wird durch Aufkleben eines 1-2 mm breiten Klebestreifens (Tesa 4124) in Position y=6 mm über die gesamte Membranbreite von der übrigen Membran separiert.

### Testansatz:

Als Blutproben werden antikoagulierte Vollblute verwendet. Für den eigentlichen Test werden 100 µl unverdünntes bzw. 1:3 bzw. 1:6 in Verdünnungspuffer (EnlisstII, Medion Diagnostics oder Diluent 1, DiaMed) verdünntes Blut in die Aufgabezone aufgetragen. Wenn das Blut die Aufgabezone verlassen hat, wird zweimalig mit 100 µl EnlisstII gewaschen, um ungebundene Erythrozyten aus der Membran zu waschen.

Im Anschluß werden 50 µl eines Gemisches aus anti-IgG/A/M konjugierten Goldpartikeln (20 bis 40 nm, Arista Biologicals, CGIGA-0800, CGIGG-0800, CGIGM-0800), 1:10 (v/v) verdünnt in TBS , 0.08 % Gelatine, 0.5 % Albumin, auf die Aufgabezone aufgetragen. Anstelle der Goldpartikel können auch farbige, 100 bis 400 nm Polystyrolpartikel verwendet werden, z.B. von Merck Eurolab France/Estapor. Wenn die Goldpartikel die Aufgabezone verlassen haben, wird die Membran nochmals ein- oder zweimalig mit 100 µl EnlisstII gewaschen.

### Ergebnis:

Der Test ist gültig, wenn die anti-RBC Kontrolle (Indikatorzone IX, Indikatorzonenbereich "Blutgruppenbestimmung") ein deutlich positives Signal (Roter Punkt) zeigt und wenn die anti-IgG/IgM-Kontrolle (Indikatorzone III, Indikatorzonenbereich "Nachweis von Antikörpern gegen thrombozytäre Antigene") charakteristisch pupurn (Goldpartikel) oder in der Farbe der verwendeten Polystyrolpartikel gefärbt ist. Je nach Anwesenheit oder Abwesenheit der jeweiligen Blutgruppenantigene erscheinen an den entsprechenden Positionen rote Punkte (positiv) oder die fast weisse Hintergrundfärbung der Membran (negativ). Bei Vorhandensein von Antikörpern gegen thrombozytäre Antigene ist die jeweilige Position durch das für Gold-Partikel charakteristische Purpur angefärbt und als purpurfarbene Spots erkennbar. Wenn Polystyrolpartikel als Indikator-Partikel verwendet werden, ist die jeweilige Position in der Farbe der verwendeten Polystyrolpartikel gefärbt.

## Patentansprüche

1. Vorrichtung zum gleichzeitigen, qualitativen oder quantitativen Bestimmen mehrerer Analyten, wobei wenigstens ein Analyt ein zellulär-gebundener Analyt ist, in einer flüssigen Probe, wobei für die Bestimmung mindestens zwei Sorten Indikatorpartikel verwendet werden, von denen mindestens eine Sorte Erythrozyten sind, umfassend:
- eine Aufgabezone (5) zum Auftragen der flüssigen Probe,
- eine zum Eindringen von zellulären Komponenten geeignete poröse Membran (2) mit mindestens zwei Indikatorzonen auf der Membran, die mit dem/den Analyten in Wechselwirkung treten können und
- mindestens einem Absorptionsbereich (3) auf der Membran, welcher die Flüssigkeit nach Passieren der Indikatorzonen aufnimmt,
wobei die Indikatorzonen zwischen der Aufgabezone (5) und einem Absorptionsbereich (3) liegen, wobei die Fließrichtungen von der Aufgabezone (5) durch die jeweiligen Indikatorzonen zu einem Absorptionsbereich (3) (Fließspuren) im Wesentlichen parallel sind und mindestens zwei unterschiedliche Fließspuren vorliegen, wobei die Membran eine erste Indikatorzone umfasst, die ein Bindungselement zur Bindung eines zellulär-gebundenen Analyten enthält und die Membran eine zweite Indikatorzone umfasst, die ein Bindungselement zur Bindung eines im Plasma enthaltenen Analyten enthält.

2. Vorrichtung nach Anspruch 1, wobei die Indikatorzonen so angeordnet sind, dass die Probenflüssigkeit pro Fließspur nicht mehr als eine Indikatorzone durchströmt.

3. Vorrichtung nach Anspruch 1, wobei die Indikatorzonen in einer diagonalen, V-, W-, M-, N-förmigen oder linearen Reihe angeordnet sind.

4. Vorrichtung nach einem der Ansprüche 1 bis 3, wobei mindestens zwei Reihen von Indikatorzonen in Fließrichtung hintereinander und/oder seitlich versetzt angeordnet sind und die Indikatorzonen der verschiedenen Reihen zueinander auf Lücke angeordnet sind, so dass die Probenflüssigkeit pro Fließspur nicht mehr als eine Indikatorzone durchströmt.

5. Vorrichtung nach Anspruch 1 oder 3, wobei mindestens zwei Reihen von Indikatorzonen in Fließrichtung hintereinander und/oder seitlich angeordnet sind und die Indikatorzonen der verschiedenen Reihen zueinander nicht auf Lücke angeordnet sind, so dass die Probenflüssigkeit pro Fließspur mehr als eine Indikatorzone durchströmt.

6. Vorrichtung nach Anspruch 1 bis 3, wobei mindestens 2 Gruppen von Indikatorzonen angeordnet sind, welche von der Aufgabezone her in unterschiedlichen Fliessrichtungen liegen.

7. Vorrichtung nach einem der Ansprüche 1 bis 6, wobei die Indikatorzonen Antikörper bzw. Antikörperfragmente bzw. Lektine, Antigene bzw. Antigen-Epitope und/oder Zellen bzw. Zellfragmente umfassen.

8. Vorrichtung nach einem der Ansprüche 1 bis 7, wobei die Indikatorzonen Antikörper bzw. Antikörper-Fragmente gegen Blutgruppenantigene bzw. -antigen-Epitope und Membranen bzw. Zellfragmente von Blutgruppe A1, A2, B und/oder 0 Erythrozyten umfassen.

9. Vorrichtung nach einem der Ansprüche 1 bis 7, wobei die Indikatorzonen Antikörper bzw. Antikörper-Fragmente gegen Blutgruppenantigene bzw. -antigen-Epitope und synthetische Peptide, rekombinante Antigene und/oder Antikörper bzw. Antikörper-Fragmente gegen infektiöse Marker umfassen.

10. Vorrichtung nach einem der Ansprüche 1 bis 7, wobei die Indikatorzonen Antikörper bzw. Antikörper-Fragmente gegen Blutgruppenantigene bzw. -antigen-Epitope und Fragmente von Thrombozyten und/oder Lymphozyten umfassen.

11. Vorrichtung nach einem der Ansprüche 1 bis 10, wobei die Membran oder die Membranen (2) aus Polyethylen, Nitrozellulose oder Nylon, bestehen.

12. Vorrichtung nach einem der Ansprüche 1 bis 11, wobei hinter der Aufgabezone (5) und vor den Indikatorzonen auf der Membran (2) mindestens ein Dichtelement (4) angeordnet ist.

13. Vorrichtung nach einem der Ansprüche 1 bis 12, wobei hinter dem Dichtelement (4) und vor den Indikatorzonen mindestens ein Konjugat-Pad aufgebracht ist.

14. Vorrichtung nach einem der Ansprüche 1 bis 13, wobei die Komponenten der Vorrichtung zur mechanischen Verstärkung auf eine Trägerschicht (1) aufgebracht sind.

15. Vorrichtung nach einem der Ansprüche 1 bis 14, wobei die Komponenten der Vorrichtung in einem Gehäuse integriert sind.

16. Verwendung der Vorrichtung nach einem der Ansprüche 1 bis 11 zur Analyse von Blut.

17. Verwendung nach Anspruch 16, wobei die Analyse eine simultane Durchführung der Blutgruppenbestimmung und Serumgegenprobe und/oder Antikörpersuch-Test ist.

18. Verwendung nach Anspruch 16, wobei die Analyse eine simultane Durchführung der Blutgruppenbestimmung und des Nachweises von infektionsserologischen Markern bzw. Fragmenten davon ist.

19. Verwendung nach Anspruch 16, wobei die Analyse eine simultane Durchführung der Blutgruppenbestimmung und des Nachweises von Antikörpern gegen Blutzellen, insbesondere anti-Thrombozyten oder anti-Lymphozyten Antikörper, bzw. jeweils Fragmenten davon ist.

20. Verfahren zur Bestimmung mehrerer Analyten oder deren Derivate in einer flüssigen Probe, umfassend:
das Auftragen der Probe auf die Aufgabezone (5) mindestens einer Membran (2) der Vorrichtung nach einem der vorangegangenen Ansprüche 1 bis 15, wobei diese Probe in ausreichender Menge vorliegt, um die Probenflüssigkeit dazu zu veranlassen, in Richtung Absorptionsbereich (3) durch die Indikatorzonen zu fließen und um die Analyten oder ihre Derivate in der Probenflüssigkeit dazu zu veranlassen, in den Indikatorzonen einen Komplex zu bilden, und wobei mindestens zwei Sorten Indikatorpartikel verwendet werden, von denen mindestens eine Sorte Erythrozyten sind.

21. Verfahren nach Anspruch 20, wobei die Analyte oder deren Derivate Blutgruppenantigene bzw. -antigen-Epitope, gegen Blutgruppenantigene gerichtete Antikörper bzw. Fragmente davon, gegen Thrombozyten- oder Leukozyten gerichtete Antikörper bzw. Fragmente davon oder gegen infektiöse Agenzien gerichtete Antikörper bzw. Fragmente davon oder Antigene infektiöser Agenzien bzw. Antigen-Epitope sind.

22. Verfahren nach Anspruch 20 oder 21, wobei die Analyte oder deren Derivate Antigene bzw. Antigen-Epitope der Blutgruppensysteme AB0, Rh und Kell umfassen.

23. Verfahren nach Anspruch 20 oder 21, wobei die Analyte oder deren Derivate Antikörper bzw. Fragmente davon gegen Thrombozyten und/oder Lymphozyten umfassen.

24. Verfahren nach Anspruch 20 oder 21, wobei die Analyte oder deren Derivate Antikörper bzw. Fragmente davon gegen bakterielle und/oder virale Agenzien bzw. virale oder bakterielle Antigene bzw. Antigen-Epitope umfassen.

25. Verfahren nach einem der Ansprüche 20 bis 24, wobei die flüssigen Proben aus Vollblut, Blutzell-Konzentrat, Serum, Plasma und/oder Testflüssigkeit, beispielsweise Kontrollserum oder Kontrollzellen, bestehen.

## Claims

1. A device for the simultaneous and qualitative or quantitative determination of a plurality of analytes, wherein at least one analyte is a cellularly bonded analyte, in a liquid sample, wherein for the determination at least two types of indicator particles are used of which at least one type being erythrocytes, comprising:
- an application zone (5) for the application of the liquid sample,
- a porous membrane (2) which facilitates the influx of cellular components with at least two indicator zones on the membrane, which are able to interact with the analyte(s) and
- at least one absorption region (3) on the membrane which takes up the liquid after having passed the indicator zones,
wherein the indicator zones are located between the application zone (5) and the absorption region (3), wherein the flow directions from the application zone (5) through the respective indicator zones towards an absorption region (3) (flow tracks) are substantially parallel and that at least two different flow tracks are present, wherein the membrane comprises a first indicator zone containing a bonding element for binding a cellularly bound analyte and the membrane comprises a second indicator zone containing a bonding element for binding an analyte contained in the plasma.

2. The device according to claim 1, wherein the indicator zones are so arranged that the test liquids for any one flow track flow through not more than one indicator zone.

3. The device according to claim 1, wherein the indicator zones are arranged in a diagonal V-, W-, M-, N-shaped or linear row.

4. The device according to any one of claims 1 to 3, wherein at least two rows of indicator zones are arranged in the flow direction one behind the other and/or laterally staggered and the indicator zones of the different rows are arranged in relation to one another with a gap there between so that the test liquid for any one flow track flows through not more than one indicator zone.

5. The device according to claim 1 or 3, wherein at least two rows of indicator zones are arranged in the flow direction one behind the other and/or side by side and the indicator zones of the different rows in relation to one another are arranged without a gap there between so that the test liquid for any one flow track passes through more than one indicator zone.

6. The device according to claim 1 to 3, wherein at least two groups of indicator zones are arranged which are disposed starting from the application zone in different flow directions.

7. The device according to any one of claims 1 to 6, wherein the indicator zones comprise antibodies or antibody fragments or lectines, antigens or antigen epitopes and/or cells or cell fragments.

8. The device according to any one of the claims 1 to 7, wherein the indicator zones comprise antibodies or antibody fragments against blood group antigens or antigen epitopes and membranes or cell fragments of blood groups A1, A2, B and/or O erythrocytes.

9. The device according to any one of claims 1 to 7, wherein the indicator zones comprise antibodies or antibody fragments against blood group antigens or antigen epitopes and synthetic peptides, recombinant antigens and/or antibodies or antibody fragments against infective markers.

10. The device according to any one of claims 1 to 7, wherein the indicator zones comprise antibodies or antibody fragments against blood group antigens or antigen epitopes and fragments of thrombocytes and/or lymphocytes.

11. The device according to any one of claims 1 to 10, wherein all the membranes (2) consist of polyethylene, nitrocellulose or nylon.

12. The device according to any one of claims 1 to 11, wherein downstream of the application zone (5) and upstream of the indicator zones at least one sealing element (4) is provided on the membrane (2).

13. The device according to any one of claims 1 to 12, wherein downstream of the sealing element (4) and upstream of the indicator zones at least one conjugate pad is applied.

14. The device according to any one of claims 1 to 13, wherein the components of the device have been applied onto a support layer (1) for mechanical reinforcement.

15. The device according to any one of claims 1 to 14, wherein the components of the device are integrated in a casing.

16. Use of the device according to any one of claims 1 to 11 for the analysis of blood.

17. Use according to claim 16, wherein the analysis is a simultaneous performance of blood group determinations and serum reverse grouping and/or antibody detection test.

18. Use according to claim 16, wherein the analysis is a simultaneous performance of blood group determinations and the detection of infection serological markers or fragments thereof.

19. Use according to claim 16, wherein the analysis is a simultaneous performance of blood group determinations and the detection of antibodies against blood cells, in particular anti-thrombocyte or ant-lymphocyte antibodies or the respective fragments thereof.

20. A process for the determination of a plurality of analytes or their derivatives in a liquid sample, comprising:
the application of the sample onto the application zone (5) of at least one membrane (2) of the device according to any one of the preceding claims 1 to 15, wherein this sample is present in adequate amounts in order to induce the test liquid to flow in the direction of the absorption region (3) through the indicator zones and to induce the analytes or their derivatives in the test liquid to form a complex in the indicator zones, wherein at least two types of indicator particles are used of which at least one type being erythrocyte.

21. The process according to claim 20, wherein the analytes or their derivatives are blood group antigens or antigen epitopes, antibodies directed against blood group antigens or fragments thereof, antibodies or fragments thereof directed against thrombocytes or leukocytes or antibodies or fragments thereof directed against infective agents or antigens of infective agents or antigen epitopes.

22. The process according to claims 20 or 21, wherein the analytes or their derivatives include antigens or antigen epitopes of the blood group systems ABO, Rh and Kell.

23. The process according to claims 20 or 21, wherein the analytes or their derivatives include antibodies or fragments thereof against thrombocytes and/or lymphocytes.

24. The process according to claims 20 or 21, wherein the analytes or their derivatives include antibodies or fragments thereof against bacterial and/or viral agents or viral or bacterial antigens or antigen epitopes.

25. The process according to any one of claims 20 to 24, wherein the liquid samples comprise whole blood, blood cell concentrate, serum, plasma and/or test liquid, for example control serum or control cells.

## Revendications

1. Dispositif pour la détermination qualitative ou quantitative simultanée de plusieurs analytes, où au moins un analyte est un analyte à liaison cellulaire, dans un échantillon liquide, au moins deux sortes de particules indicatrices étant utilisées pour la détermination, parmi lesquelles au moins une sorte est constituée d'érythrocytes, comprenant :
- une zone de dépôt (5) pour application de l'échantillon liquide,
- une membrane poreuse (2), convenant à la pénétration de composants cellulaires, comportant au moins deux zones indicatrices sur la membrane, qui peuvent entrer en interaction avec l'analyte/les analytes, et
- au moins un domaine d'absorption (3) sur la membrane, qui absorbe le liquide après que ce dernier a traversé les zones indicatrices,
dans lequel les zones indicatrices sont situées entre la zone de dépôt (5) et un domaine d'absorption (3), les directions d'écoulement allant de la zone de dépôt (5) à un domaine d'absorption (3) (traces d'écoulement) en passant par les zones indicatrices correspondantes, étant pour l'essentiel parallèles, et au moins deux traces d'écoulement différentes étant présentes, la membrane comprenant une première zone indicatrice qui contient un élément de liaison pour la liaison d'un analyte à liaison cellulaire, et la membrane comprenant une deuxième zone indicatrice, qui contient un élément de liaison pour la liaison d'un analyte contenu dans le plasma.

2. Dispositif selon la revendication 1, dans lequel les zones indicatrices sont disposées de telle sorte que le liquide échantillon ne traverse, par trace d'écoulement, pas plus d'une zone indicatrice.

3. Dispositif selon la revendication 1, dans lequel les zones indicatrices sont disposées selon une rangée diagonale, en forme de v, de W, de M, de N, ou linéaire.

4. Dispositif selon l'une des revendications 1 à 3, dans lequel au moins deux rangées de zones indicatrices sont disposées les unes derrière les autres dans la direction de l'écoulement, et/ou en décalage latéral, et les zones indicatrices des différentes rangées sont disposées en chicane l'une par rapport à l'autre, de telle sorte que le liquide échantillon ne traverse, par trace d'écoulement, pas plus d'une zone indicatrice.

5. Dispositif selon la revendication 1 ou 3, dans lequel au moins deux rangées de zones indicatrices sont disposées l'une derrière l'autre dans la direction de l'écoulement, et/ou latéralement, et les zones indicatrices des différentes rangées ne sont pas disposées en chicane l'une par rapport à l'autre, de sorte que le liquide échantillon ne traverse, par trace d'écoulement, pas plus d'une zone indicatrice.

6. Dispositif selon les revendications 1 à 3, dans lequel au moins deux groupes de zones indicatrices sont disposées, qui, en partant de la zone de dépôt, se trouvent dans des directions d'écoulement différentes.

7. Dispositif selon l'une des revendications 1 à 6, dans lequel les zones indicatrices comprennent des anticorps ou des fragments d'anticorps ou des lectines, des antigènes ou des épitopes antigéniques et/ou des cellules ou des fragments cellulaires.

8. Dispositif selon l'une des revendications 1 à 7, dans lequel les zones indicatrices comprennent des anticorps ou des fragments d'anticorps contre des antigènes ou épitopes antigéniques de groupes sanguins, et des membranes ou des fragments cellulaires d'érythrocytes des groupes sanguins A1, A2, B et/ou O.

9. Dispositif selon l'une des revendications 1 à 7, dans lequel les zones indicatrices comprennent des anticorps ou des fragments d'anticorps contre des antigènes ou épitopes antigéniques de groupes sanguins et des peptides synthétiques, des antigènes recombinants, et/ou des anticorps ou des fragments d'anticorps contre des marqueurs infectieux.

10. Dispositif selon l'une des revendications 1 à 7, dans lequel les zones indicatrices comprennent des anticorps ou des fragments d'anticorps contre des antigènes ou des épitopes antigéniques de groupes sanguins, et des fragments de thrombocytes et/ou de lymphocytes.

11. Dispositif selon l'une des revendications 1 à 10, dans lequel la membrane ou les membranes (2) sont constituées de polyéthylène, de nitrocellulose ou de nylon.

12. Dispositif selon l'une des revendications 1 à 11, dans lequel au moins un élément d'étanchéité (4) est disposé en arrière de la zone de dépôt (5), et en avant des zones indicatrices sur la membrane (2).

13. Dispositif selon l'une des revendications 1 à 12, dans lequel on a disposé au moins une zone de dépôt de conjugués en arrière de l'élément d'étanchéité (4) et en avant des zones indicatrices.

14. Dispositif selon l'une des revendications 1 à 13, dans lequel les composants du dispositif sont, pour un renforcement mécanique, disposés sur une couche support (1).

15. Dispositif selon l'une des revendications 1 à 14, dans lequel les composants du dispositif sont intégrés dans un boîtier.

16. Utilisation du dispositif selon l'une des revendications 1 à 11 pour l'analyse du sang.

17. Utilisation selon la revendication 16, pour laquelle l'analyse représente une opération simultanée de détermination du groupe sanguin et un test de contrôle du sérum et/ou un test de recherche d'anticorps.

18. Utilisation selon la revendication 16, pour laquelle l'analyse est une opération simultanée de détermination du groupe sanguin et de détection de marqueurs sérologiques infectieux ou de fragments de ceux-ci.

19. Utilisation selon la revendication 16, pour laquelle l'analyse est une opération simultanée de détermination du groupe sanguin et de détection d'anticorps contre des globules sanguins, en particulier des anticorps anti-thrombocytes ou anti-lymphocytes, ou des fragments de chacun d'eux.

20. Procédé de détermination de plusieurs analytes ou de leurs dérivés dans un échantillon liquide, comprenant :
l'application de l'échantillon sur la zone de dépôt (5) d'au moins une membrane (2) du dispositif selon l'une des revendications 1 à 15 ci-dessus, où cet échantillon est présent en une quantité suffisante pour amener l'échantillon liquide à s'écouler dans la direction du domaine d'absorption (3) en passant par les zones indicatrices, et pour amener les analytes ou leurs dérivés se trouvant dans le liquide échantillon à former un complexe dans les zones indicatrices, au moins deux sortes de particules indicatrices étant utilisées, dont au moins l'une est constituée d'érythrocytes.

21. Procédé selon la revendication 20, dans lequel les analytes ou leurs dérivés sont des antigènes ou des épitopes antigéniques de groupes sanguins, des anticorps dirigés contre des antigènes de groupes sanguins ou des fragments de ceux-ci, des anticorps dirigés contre des thrombocytes ou des leucocytes ou des fragments de ceux-ci, ou des anticorps dirigés contre des agents infectieux ou des fragments de ceux-ci, ou des antigènes d'agents infectieux, ou des épitopes antigéniques.

22. Procédé selon la revendication 20 ou 21, dans lequel les analytes ou leurs dérivés comprennent des antigènes ou des épitopes antigéniques des systèmes de groupes sanguins A, B, O, Rh et Kell.

23. Procédé selon la revendication 20 ou 21, dans lequel les analytes ou leurs dérivés comprennent des anticorps ou des fragments de ceux-ci contre des thrombocytes et/ou des lymphocytes.

24. Procédé selon la revendication 20 ou 21, dans lequel les analytes ou leurs dérivés comprennent des anticorps ou des fragments de ceux-ci contre des agents bactériens et/ou viraux ou des agents viraux ou bactériens ou des épitopes antigéniques.

25. Procédé selon l'une des revendications 20 à 24, dans lequel les échantillons liquides sont constitués de sang total, de concentré de globules sanguins, de sérum, de plasma et/ou d'un liquide d'essai, par exemple un sérum témoin ou des cellules témoins.
